# EUROPEAN PATENT APPLICATION

(11) **EP 4 609 872 A1**
(43) Date of publication of application: **03.09.2025**
(21) Application number: 25160139.9
(22) Date of filing: 26.02.2025
(51) Int. Cl.: A61K 35/741, A61K 35/744, A61K 35/745, A61K 35/747, A61K 35/00, A61P 1/00, A61P 1/10, A61P 1/12

(54) **PHARMACEUTICAL COMPOSITION FOR TREATING IBS**

(30) Priority: 28.02.2024 EP 24160123
(71) Applicant: Pronaos s.r.o., 130 00 Prague 3 (CZ)
(72) Inventor: SOMMERMEYER, Henning, 10100 Praha (CZ)
(74) Representative: Isarpatent

(57) **Abstract**

The present invention provides a pharmaceutical composition for treating IBS.

## Description

### Field of the invention

The present invention relates to a pharmaceutical composition for treating irritable bowel syndrome (IBS).

### Background of the Invention

Irritable bowel syndrome (IBS) is a common and chronic functional gastrointestinal disorder (FGID) characterized by recurrent abdominal pain, bloating, and changes in stool form and frequency [1]. IBS is a non-fatal condition but the troublesome and unpredictable character of IBS symptoms have a negative impact on patients' quality of life and work productivity [2, 3]. The consistent health concerns of IBS patients can lead to anxiety and depression [4]. The disease results in increased healthcare utilization and healthcare costs [5, 6]. Diagnosis of IBS is based solely on patients reported symptoms and the exclusion of other organic diseases as no reliable biological marker of IBS has yet been identified [7]. IBS can be subtyped by severity [8, 9] and by predominant stool pattern assessed by using the Bristol Stool Form Scale [10]: IBS with diarrhea (IBS-D), IBS with constipation (IBS-C), mixed IBS (IBS-M) or untyped IBS (IBS-U). For the latter there is insufficient abnormality of stool consistency to meet criteria for IBS-C, D, or M. The pooled prevalence of IBS in the general population is about 11.2% [11], however varies substantially among regions and diagnostic criteria applied [2]. IBS manifests during adolescence with females being more frequently affected than males [12].

The etiology of IBS is not fully understood but the condition is assumed to be multifactorial with environmental, inherited and psychosocial factor contributing to disease manifestation. Suggested mechanisms include visceral hypersensitivity, dysfunction of the gut-brain axis, disturbances of the integrity of the gut epithelial barrier leading to an abnormal intestinal permeability, altered gastrointestinal motility, activation of immune system, disturbance of the enteroendocrine signaling, and dysbiosis of the gut microbiota [13, 14]. Alteration of the gut microbiome as a potential cause or contributor to IBS has caught rising interest as those of IBS patients differ from those of healthy controls [15-17] and specific gut microbiota profiles have been associated with particular symptoms and severity of diseases [18, 19]. Recent years has seen a steady increase in the number of publications reporting results from clinical trials in IBS patients investigating the effects of probiotics, prebiotics and synbiotics [20].

As the gut microbiota seems to play a crucial role in IBS, altering the composition of the gut microbiota of IBS patients by supplementation with products containing probiotic bacteria has become a potential strategy for IBS management. Numerous single-strain and multi-strain probiotics or synbiotics (products containing probiotic bacteria and a prebiotic component) have been evaluated in randomized clinical trials. As with conventional drug interventions, treatment of IBS patients with probiotics have been shown to improve at least some of the IBS symptoms [21]. A number of recent meta-analyses and systematic reviews has evaluated the effects of products containing probiotic bacteria in IBS patients [20, 22-28]. However, determining the therapeutic value of these products is inherently complex, due to the large diversity of studied products (mono-, multi-strain products, probiotics, synbiotics), the variety of study designs and the often small number of patients investigated in the trials.

While results of these studies should be interpreted with care, at least some mono-strain and multi-strain products seems to have beneficial symptom improving effects in IBS patients. Results also revealed that the benefits provided by probiotics are strain-specific rather than species-specific. While some analyses have found that multi-strain products are more effective than mono-strain products [22, 26] others found no superiority [23, 24]. The relatively limited number of clinical trials investigating synbiotic therapies in IBS patients have thus far produced varying results which could be due to the different probiotic and prebiotic components used in different trials as well as the different subsets of IBS patient studied [27, 29, 30].

While individual probiotic bacteria and certain mixtures of probiotic bacteria in combination with or without a prebiotic component have been demonstrated to improve some symptomatic aspects of IBS, so far, no individual probiotic bacteria or mixture of probiotic bacteria have been demonstrated to exhibit a broad activity resulting in improvements of all major symptoms occurring in IBS patients.

Thus, it is an object underlying the present invention to provide a pharmaceutical composition capable of improving all major symptoms occurring in IBS patients. Furthermore, it is an object of the present invention to provide a pharmaceutical composition capable of improving symptoms in patients with severe or moderate IBS and/or IBS-D and/or IBS-C. In addition, it is an object of the present invention to provide a pharmaceutical composition which can be used in the treatment of severe or moderate IBS and/or IBS-D and/or IBS-C.

### Summary of the invention

The present invention provides a pharmaceutical composition for use in a method for the treatment of irritable bowel syndrome (IBS). The pharmaceutical composition comprises a probiotic comprising or consisting of the bacterial strains Lactobacillus helveticus, Lacticaseibacillus rhamnosus, Lacticaseibacillus casei, Lactiplantibacillus plantarum, Lactococcus lactis, Bifidobacterium longum, Bifidobacterium bifidum, Streptococcus thermophilus and optionally Bifidobacterium breve. The inventors surprisingly found that the treatment of IBS patients with the pharmaceutical composition leads to an improvement of all major symptoms associated with IBS. Furthermore, the inventors have found that the pharmaceutical composition can be successfully used for the treatment of IBS-C, IBS-D as well as for the treatment of severe or moderate IBS.

Further aspects and embodiments of the invention are disclosed in the dependent claims and can be taken from the following description, figures and examples, without being limited thereto.

### Figures

The enclosed drawings should illustrate embodiments of the present invention and convey a further understanding thereof. In connection with the description, they serve as explanation of concepts and principles of the invention. Other embodiments and many of the stated advantages can be derived in relation to the drawings.

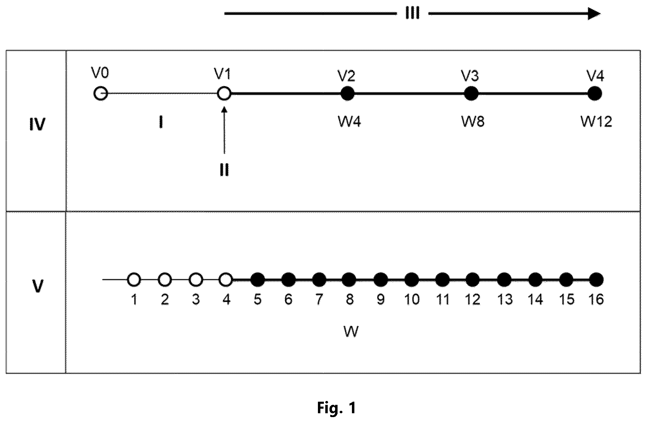

Figure 1 shows a summary of the study design. Patient were enrolled at Visit 0 (V0) to the trial. After a treatment-free run-in phase of 4 weeks (I) patients were randomized (1:1) to a placebo or a multi-strain synbiotic group (II) during visit 1 (V1). Treatment was for a total of 12 weeks (III), with examinations by physicians every four weeks (V2, V3, and V4). Physicians reported their measurements (V0 to V4) in a Physician Data Log Book (IV) and patients reported weekly symptoms in a Patient Diary (V). Abbreviations: V=visit, W=week.

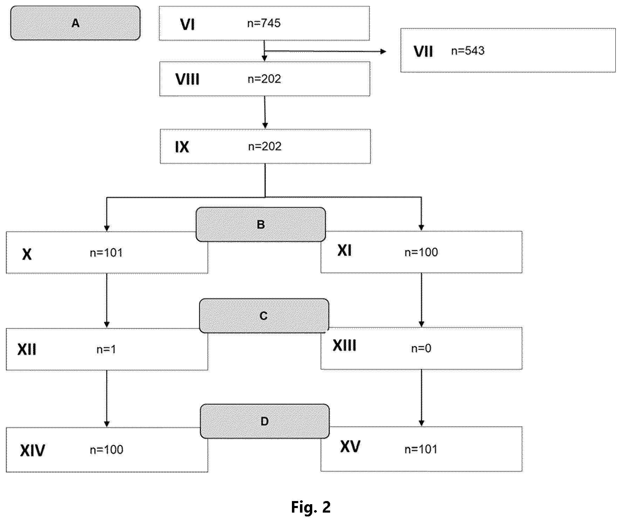

Figure 2 shows the patient enrollment and study progress. The study was a placebo-controlled, randomized, double-blind, multicentric clinical trial in primary care IBS patients. A: enrollment, B: allocation, C: follow-up, D: analysis. VI: assessment for eligibility; VII: patients excluded, not meeting inclusion criteria (no IBS) n=417; meeting exclusion criteria n=37, declined to participate n=89; VIII: IBS patients enrolled; IX: randomization; X: patient allocated to treatment with placebo; XI: patients allocated to treatment with multi-strain synbiotic, XII: patients taking placebo lost in follow-up; XIII: patients taking multi-strain synbiotic lost in follow-up; XIV: patients taking placebo analyzed; XV: patients taking multi-strain synbiotic analyzed. Abbreviation: n = number of patients.

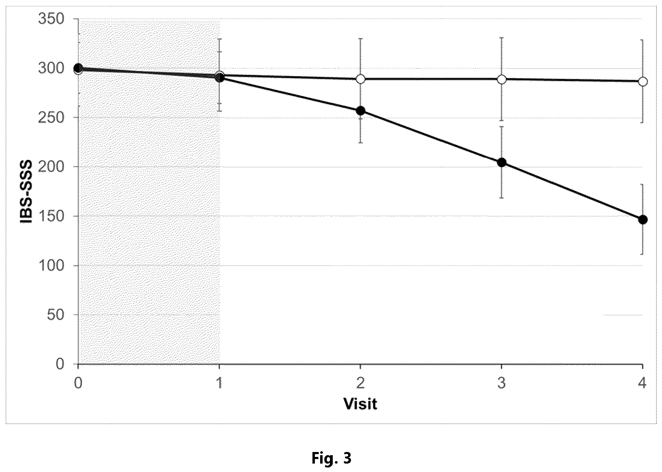

Figure 3 shows the change of IBS-severity assessed with the Irritable Bowel Syndrome - Severity of Symptom Scale (IBS-SSS). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS score. Data shown are mean ± standard deviation. Open circles represent measures for the placebo group (n=100), filled circles represent patients of the multi-strain synbiotic treatment group (n=101).

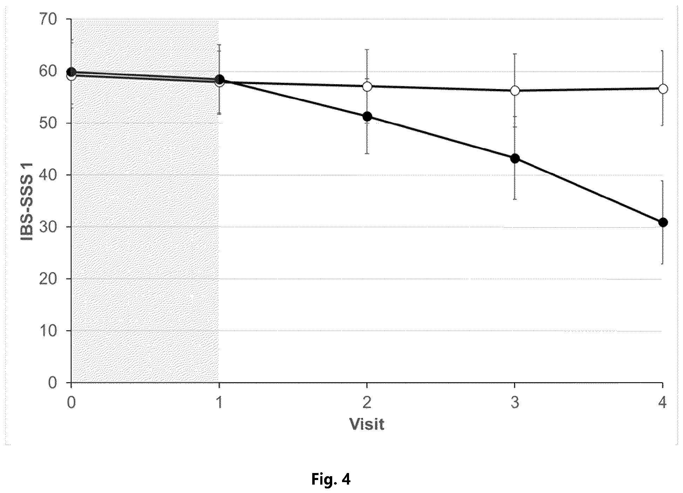

Figure 4 shows the change of the severity of abdominal pain assessed with the Irritable Bowel Syndrome - Severity of Symptom Subscale 1 (IBS-SSS1) score. On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS1 score. Data shown are mean ± standard deviation. Open circles represent measures for the placebo group (n=100), filled circles represent patients of the multi-strain synbiotic treatment group (n=101).

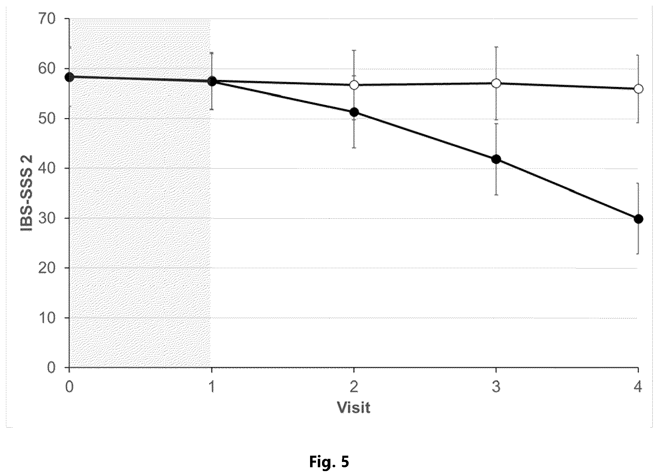

Figure 5 shows the change of the frequency of abdominal pain assessed with the Irritable Bowel Syndrome - Severity of Symptom Subscale 2 (IBS-SSS2) score. On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS2 score. Data shown are mean ± standard deviation. Open circles represent measures for the placebo group (n=100), filled circles represent patients of the multi-strain synbiotic treatment group (n=101).

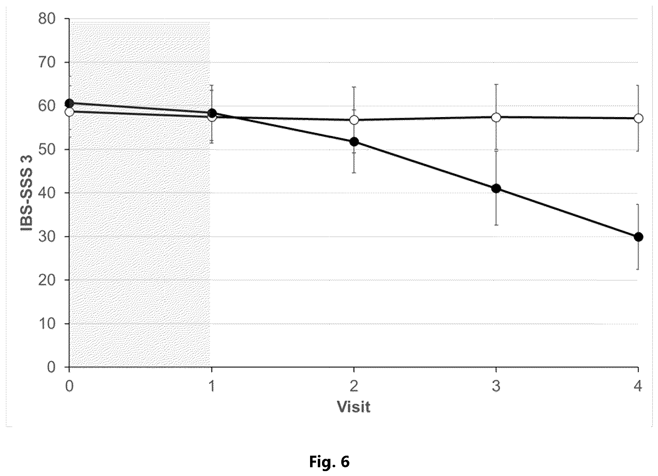

Figure 6 shows the change of the severity of flatulence assessed with the Irritable Bowel Syndrome - Severity of Symptom Subscale 3 (IBS-SSS3). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS3 score. Data shown are mean ± standard deviation. Open circles represent measures for the placebo group (n=100), filled circles represent patients of the multi-strain synbiotic treatment group (n=101).

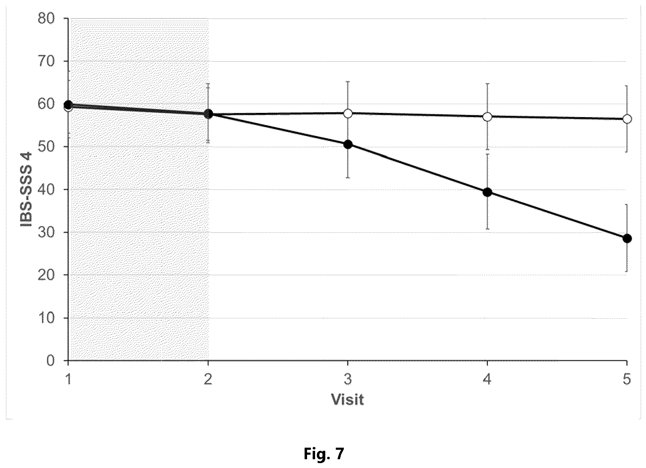

Figure 7 shows the change of dissatisfaction with bowel habit assessed with the Irritable Bowel Syndrome - Severity of Symptom Subscale 4 (IBS-SSS4). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS4 score. Data shown are mean ± standard deviation. Open circles represent measures for the placebo group (n=100), filled circles represent patients of the multi-strain synbiotic treatment group (n=101).

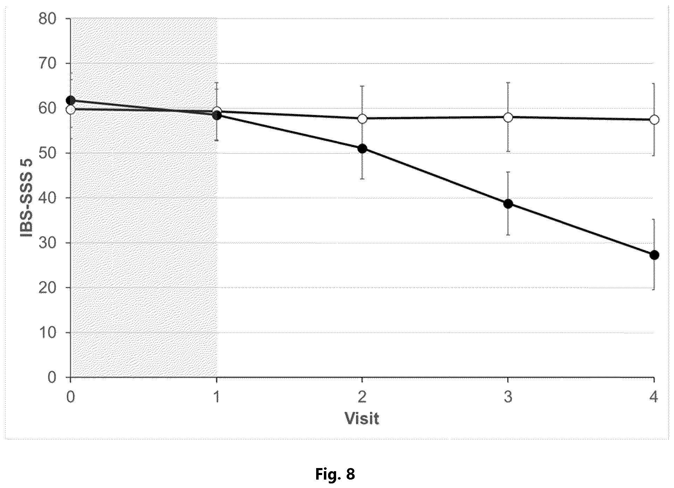

Figure 8 shows the change of interference with quality of life assessed with the Irritable Bowel Syndrome - Severity of Symptom Subscale 5 (IBS-SSS5). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS5 score. Data shown are mean ± standard deviation. Open circles represent measures for the placebo group (n=100), filled circles represent patients of the multi-strain synbiotic treatment group (n=101).

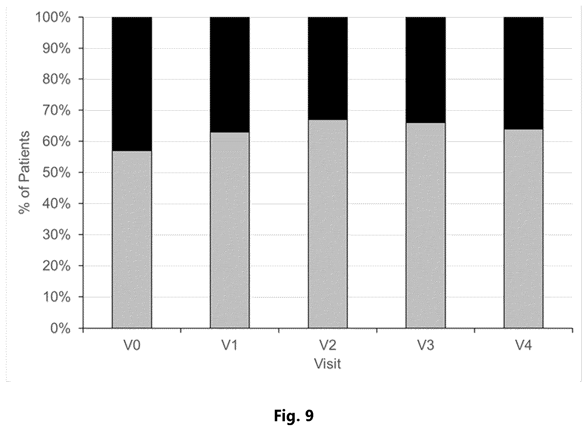

Figure 9 shows the relative portion of patients with a certain IBS-severity determined in the placebo group of patients (n=100). IBS severity was determined by using the IBS-SSS scale during each visit (V0 to V4). Individual IBS severity categories were determined by using IBS-SSS <175 for mild (white part of the bar), 175-300 for moderate (grey part of the bar), and >300 for severe (black part of the bar) IBS severity.

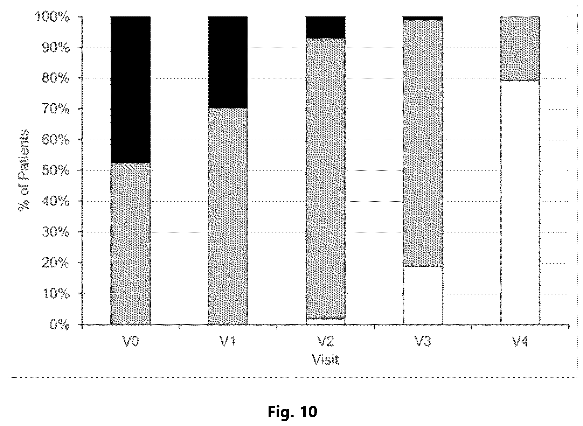

Figure 10 shows the relative portion of patients with a certain IBS-severity determined in the multi-strain synbiotic group of patients (n=101). IBS severity was determined by using the IBS-SSS scale during each visit (V0 to V4). Individual IBS severity categories were determined by using IBS-SSS <175 for mild (white part of the bar), 175-300 for moderate (grey part of the bar), and >300 for severe (black part of the bar) IBS severity.

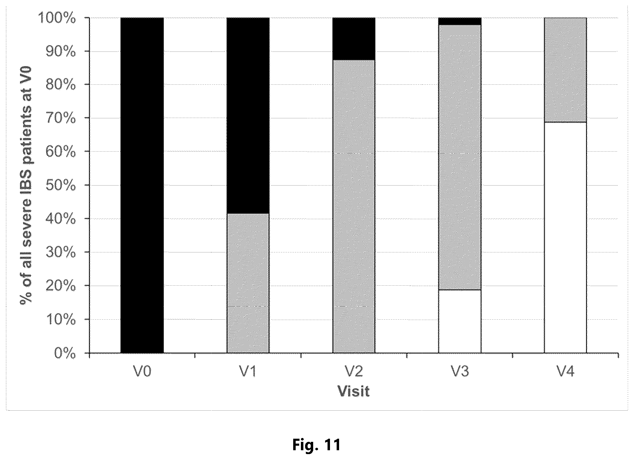

Figure 11 shows the relative portion of patients with a certain IBS-severity determined in the multi-strain synbiotic group of patients with severe IBS at visit 0 (V0) (n = 48). IBS severity was determined by using the IBS-SSS scale during each visit (V0 to V4). Individual IBS severity categories were determined by using IBS-SSS <175 for mild (white part of the bar), 175-300 for moderate (grey part of the bar), and >300 for severe (black part of the bar) IBS severity.

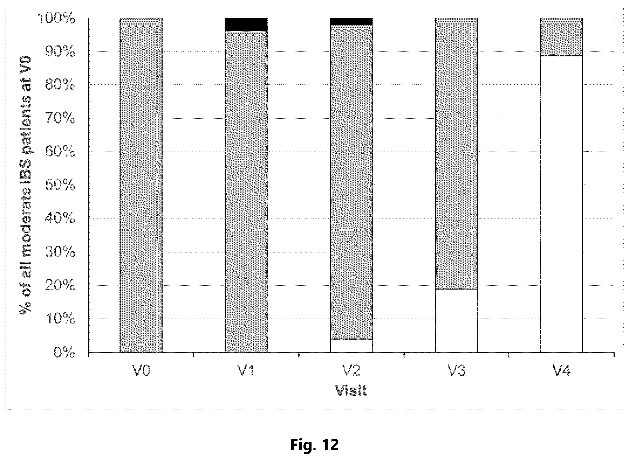

Figure 12 shows the relative portion of patients with a certain IBS-severity determined in the multi-strain synbiotic group of patients with moderate IBS at visit 0 (V0) (n = 53). IBS severity was determined by using the IBS-SSS scale during each visit (V0 to V4). Individual IBS severity categories were determined by using IBS-SSS <175 for mild (white part of the bar), 175-300 for moderate (grey part of the bar), and >300 for severe (black part of the bar) IBS severity.

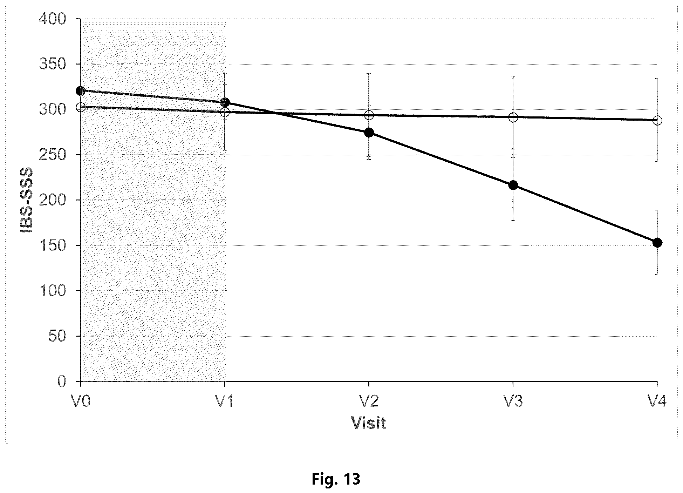

Figure 13 shows the change of IBS-severity assessed with the Irritable Bowel Syndrome - Severity of Symptom Scale (IBS-SSS) in patients with severe IBS at visit 0 (V0). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS score. Data shown are mean ± standard deviation. Open circles represent measures for the placebo group (n=43), filled circles represent patients of the multi-strain synbiotic treatment group (n=48).

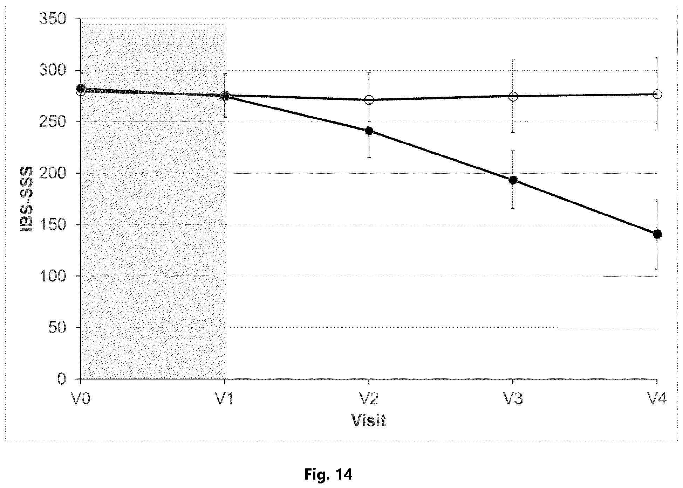

Figure 14 shows the change of IBS-severity assessed with the Irritable Bowel Syndrome - Severity of Symptom Scale (IBS-SSS) in patients with moderate IBS at visit 0 (V0). On the x-axis the respective trial visit number is shown (0: enrollment, 1: end of the 4 weeks run-in phase; 2: visit after 4 weeks of treatment, 3: visit after 8 weeks of treatment, 4: last trial visit after 12 weeks of treatment. The 4 weeks treatment-free run-in phase (visit 0 to 1) is highlighted in light grey. The y-axis shows the IBS-SSS score. Data shown are mean ± standard deviation. Open circles represent measures for the placebo group (n=57), filled circles represent patients of the multi-strain synbiotic treatment group (n=53).

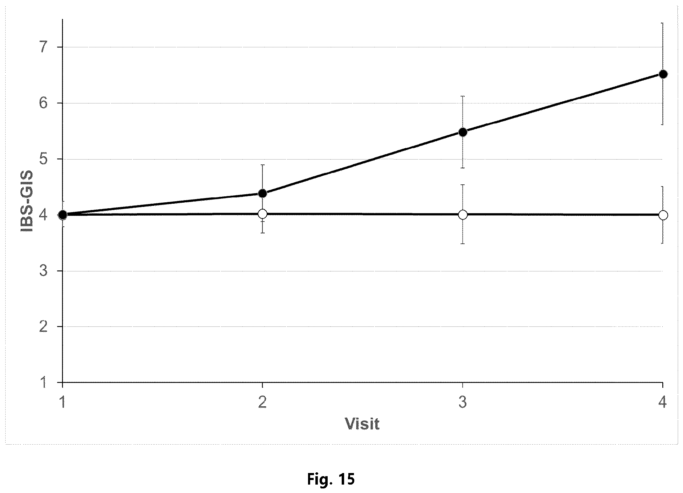

Figure 15 shows the global improvement of IBS symptoms assessed with the Irritable Bowel Syndrome - Global Improvement Scale (IBS-GIS). On the x-axis the respective trial visit number is shown (1: end of the 4 weeks run-in phase and start of treatment; 2: visit after 4 weeks of treatment, 3: visit after 8 weeks of treatment, 4: last trial visit after 12 weeks of treatment. The y-axis shows the IBS-GIS score ranging from 1 for significant worsening to 7 for significant improvement with 4 indicating no change. Data shown are mean ± standard deviation. Open circles represent measures for the placebo group (n=100), filled circles represent patients of the multi-strain synbiotic treatment group (n=101).

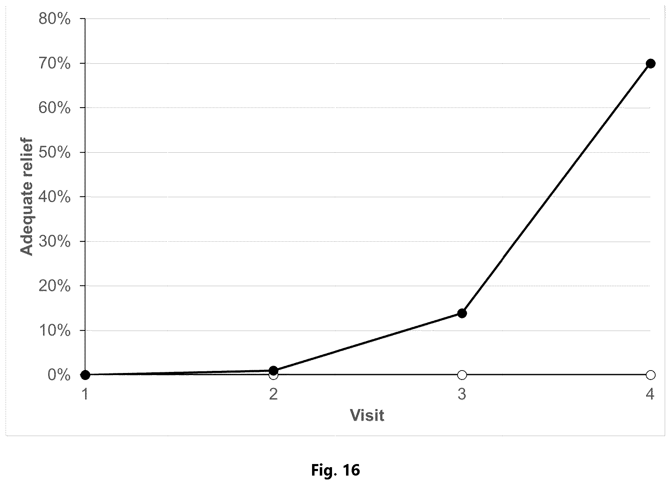

Figure 16 shows the percentage of patients with adequate relief of symptoms assessed with the Iritable Bowel Syndrome - Adequate Relief (IBS-AR) Scale as function of treatment duration. On the x-axis the respective trial visit number is shown (1: end of the 4 weeks run-in phase and start of treatment; 2: visit after 4 weeks of treatment, 3: visit after 8 weeks of treatment, 4: last trial visit after 12 weeks of treatment. The y-axis shows the percentage of patients that achieved adequate relief. Open circles represent measures for the placebo group (n=100), filled circles represent patients of the multi-strain synbiotic treatment group (n=101).

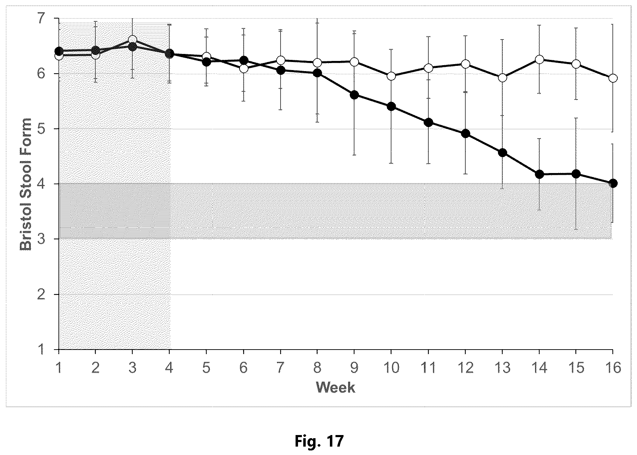

Figure 17 shows the change of stool consistency self-reported by patients with IBS-D at the beginning of the trial. Stool consistency was determined with the Bristol Stool Form Scale by patients on a weekly basis (week 1 to 16) reported in a patient diary. Randomization resulted in 73 patients in the placebo group (open circles) and 73 patients in the multi-strain synbiotic group (filled circles) having IBS-D. The 4 weeks treatment-free run-in phase (visit 1 to 4) is highlighted in light grey. After randomization at the end of week 4 patients were treated with placebo or the multi-strain synbiotic for a total of 12 weeks. Bristol Stool Form Scale scores 5-7 indicate diarrhea, 1-2 constipation and 3-4 normal stool (indicated as dark grey). Data shown are means ± standard deviation.

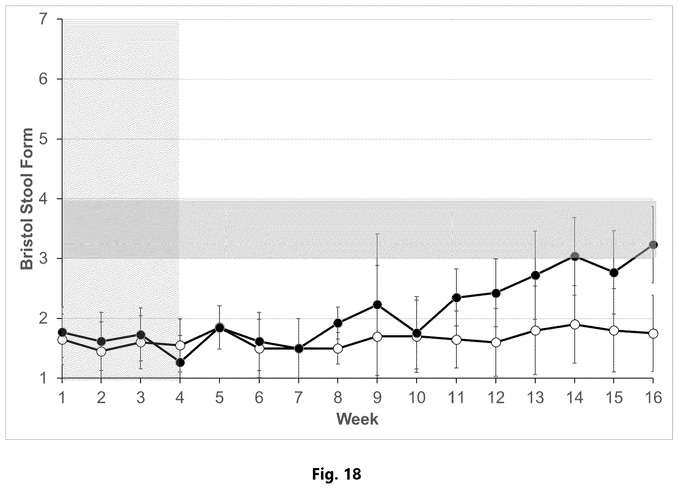

Figure 18 shows the change of stool consistency self-reported by patients with IBS-C at the beginning of the trial. Stool consistency was determined with the Bristol Stool Form Scale by patients on a weekly basis (week 1 to 16) reported in a patient diary. Randomization resulted in 20 patients in the placebo group (open circles) and 26 patients in the multi-strain synbiotic group (filled circles) having IBS-C. The 4 weeks treatment-free run-in phase (visit 1 to 4) is highlighted in light grey. After randomization at the end of week 4 patients were treated with placebo or the multi-strain synbiotic for a total of 12 weeks. Bristol Stool Form Scale scores 5-7 indicate diarrhea, 1-2 constipation and 3-4 normal stool (indicated as dark grey). Data shown are means ± standard deviation.

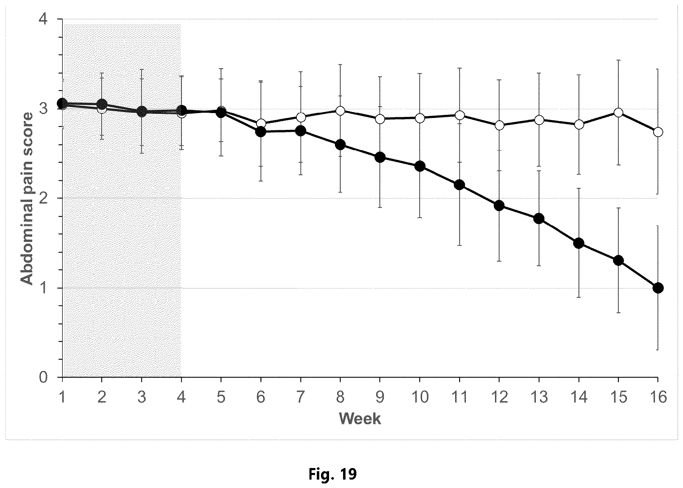

Figure 19 shows the abdominal pain severity self-reported by patients on a 5-point Likert scale in a patient diary. Randomization resulted in 100 patients in the placebo group (open circles) and 101 patients in the multi-strain synbiotic group (filled circles). The 4 weeks treatment-free run-in phase (visit 1 to 4) is highlighted in light grey. After randomization at the end of week 4 patients were treated with placebo or the multi-strain synbiotic for a total of 12 weeks. Scores on the y-axis range from 0 for no pain to 4 for very severe pain. Data shown are means ± standard deviation.

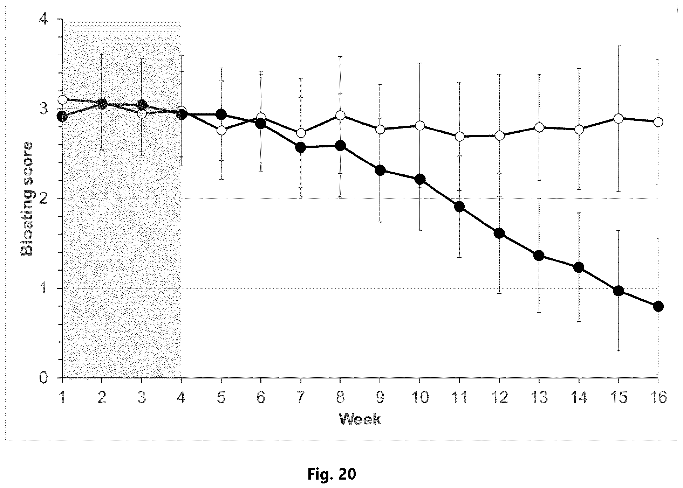

Figure 20 shows the severity of bloating self-reported by patients on a 5-point Likert scale in a patient diary. Randomization resulted in 100 patients in the placebo group (open circles) and 101 patients in the multi-strain synbiotic group (filled circles). The 4 weeks treatment-free run-in phase (visit 1 to 4) is highlighted in light grey. After randomization at the end of week 4 patients were treated with placebo or the multi-strain synbiotic for a total of 12 weeks. Scores on the y-axis range from 0 for no bloating to 4 for very severe bloating. Data shown are means ± standard deviation.

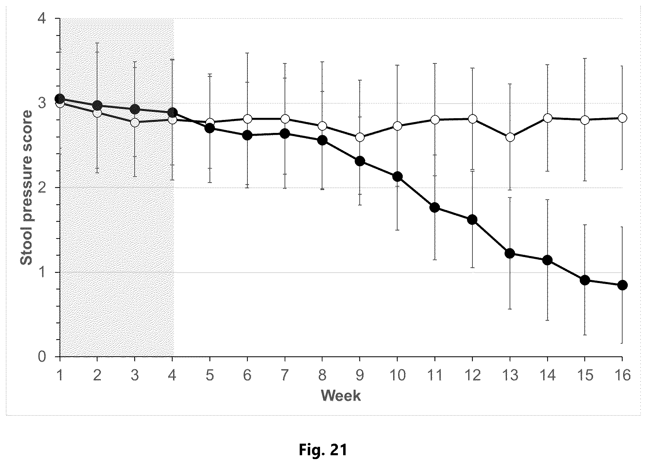

Figure 21 shows the stool pressure self-reported by patients on a 5-point Likert scale in a patient diary. Randomization resulted in 100 patients in the placebo group (open circles) and 101 patients in the multi-strain synbiotic group (filled circles). The 4 weeks treatment-free run-in phase (visit 1 to 4) is highlighted in light grey. After randomization at the end of week 4 patients were treated with placebo or the multi-strain synbiotic for a total of 12 weeks. Scores on the y-axis range from 0 for no stool pressure to 4 for very severe stool pressure. Data shown are means ± standard deviation.

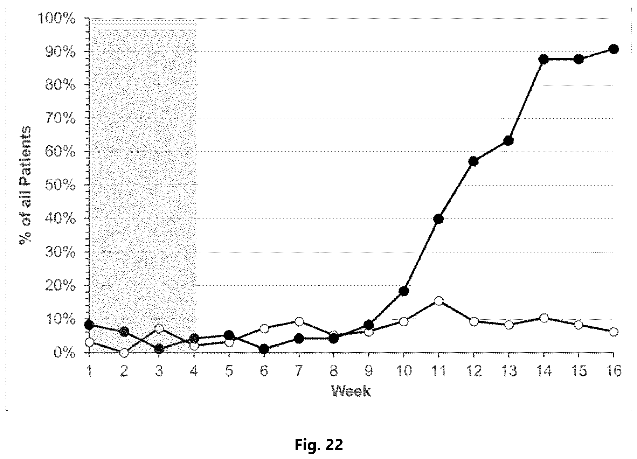

Figure 22 shows the assessment of feeling of complete stool evacuation self-reported by patients on a dichotomous ("yes" or "no") scale in a patient diary. Randomization resulted in 100 patients in the placebo group (open circles) and 101 patients in the multi-strain synbiotic group (filled circles). The 4 weeks treatment-free run-in phase (visit 1 to 4) is highlighted in light grey. After randomization at the end of week 4 patients were treated with placebo or the multi-strain synbiotic for a total of 12 weeks.

### Detailed description of the present invention

### Definitions

Unless defined otherwise, technical and scientific terms used herein have the same meaning as commonly understood by one of ordinary skill in the art to which this invention belongs.

Amounts within the present invention are given in wt.%, unless stated otherwise or clear from context.

In the context of the present invention, the term "pharmaceutical composition" is not particularly limited. As is generally understood a pharmaceutical composition refers to a formulation that includes an active ingredient (or ingredients) intended for use in the diagnosis, cure, mitigation, treatment, or prevention of disease, and which is intended to affect the structure or any function of the body of humans or animals. The pharmaceutical composition may also include various other components, such as carriers, excipients, or adjuvants, which help in the delivery, stability, absorption, or effectiveness of the active ingredient(s). Pharmaceutical compositions can take various forms, including tablets, capsules, liquids, powders, gels, ointments, and injectables, among others. The choice of form and components depends on the route of administration (oral, topical, intravenous, etc.), the nature of the active ingredient, the intended use, and the target patient population. In the present case, the terms "pharmaceutical composition" and "multi-strain synbiotic" are used synonymously.

In the context of the present invention, the abbreviation IBS stands for irritable bowel syndrome. Irritable bowel syndrome is delineated as a multifaceted gastrointestinal disorder predominantly affecting the intestine, marked by a constellation of concurrent symptoms including, but not limited to, recurrent abdominal pain or discomfort, and changes in stool form and frequency, absent any discernible organic damage.

In the context of the present invention, the abbreviation IBS-D stands for irritable bowel syndrome with diarrhea. Irritable bowel syndrome with diarrhea is a subtype of irritable bowel syndrome where the predominant symptom is frequent, loose, or watery stools. In the context of the present invention, IBS-D is present when IBS patients have a Bristol Stool Form Scale score of 5, 6 or 7, in particular over a period of at least 4 weeks. More specifically, IBS-D is present when IBS patients report a daily Bristol Stool Form Scale score of 5, 6 or 7 over a period of at least 4 weeks. The assessment of predominant stool pattern by the Bristol stool score is for example described in reference [10].

In the context of the present invention, the abbreviation IBS-C stands for irritable bowel syndrome with constipation. Irritable bowel syndrome with constipation is a subtype of irritable bowel syndrome characterized primarily by infrequent, hard, or lumpy stools. In the context of the present invention, IBS-C is present when IBS patients have a Bristol Stool Form Scale score of 1 or 2, in particular over a period of at least 4 weeks. More specifically, IBS-C is present when IBS patients report a daily Bristol stool score of 1 or 2 over a period of at least 4 weeks. The assessment of predominant stool pattern by the Bristol stool score is for example described in reference [10].

In the context of the present invention, moderate IBS is present when IBS patients have a total score between 175 and 300 on the IBS Severity of Symptom Scale (IBS-SSS). As is generally understood, moderate irritable bowel syndrome (IBS) can be quantitatively assessed using the IBS-SSS developed by Francis et al. [8]. The IBS-SSS is a validated questionnaire that measures the severity of symptoms associated with IBS, providing a comprehensive score based on five main criteria: abdominal pain severity, abdominal pain frequency, severity of abdominal flatulence, dissatisfaction with bowel habit, and interference with quality of life.

In the context of the present invention, mild IBS is present when IBS patients have a total score between below 175 on the IBS Severity of Symptom Scale (IBS-SSS). As is generally understood, mild irritable bowel syndrome (IBS) can be quantitatively assessed using the IBS-SSS developed by Francis et al. [8]. The IBS-SSS is a validated questionnaire that measures the severity of symptoms associated with IBS, providing a comprehensive score based on five main criteria: abdominal pain severity, abdominal pain frequency, severity of abdominal flatulence, dissatisfaction with bowel habit, and interference with quality of life.

In the context of the present invention, the term "probiotic" is not particularly limited. As is generally understood, a probiotic is a live microorganism, typically certain strains of bacteria or yeast, that when administered in adequate amounts, confers a health benefit on the host. Probiotics are often found in fermented foods like yogurt, kefir, sauerkraut, and kimchi, or taken as dietary supplements. They are used to improve or restore the gut microbiota for the benefit of gut health, and have been associated with a variety of health benefits, including enhancement of the immune system, reduction of gastrointestinal discomfort, and potential protection against certain infections.

In the context of the present invention, the term "prebiotic" is not particularly limited. As is generally understood, a prebiotic is a non-digestible food component that promotes the growth and activity of beneficial microorganisms in the gut. Prebiotics are typically dietary fibers or complex carbohydrates that the human body cannot digest, but that serve as food for probiotics and other beneficial bacteria in the digestive system. By supporting the growth of healthy bacteria, prebiotics contribute to the improvement of gut health, enhancement of digestion, and may also have positive effects on overall health.

In the context of the present invention, the term "synbiotic" is not particularly limited. As is generally understood, a synbiotic is a combination of at least one probiotic and at least one prebiotic that are specifically formulated to work synergistically to enhance the survival and colonization of beneficial microorganisms in the gut. The prebiotic component serves as food for the probiotic bacteria, helping to ensure their survival, growth, and activity in the digestive system. Synbiotics aim to improve the host's health by balancing the gut microbiota, enhancing digestive health, and potentially providing other health benefits related to immune function and nutrient absorption.

In the context of the present invention, the term "enteric coating" is not particularly limited. As is generally understood, an enteric coating is a polymer barrier applied to oral medication that prevents its dissolution or disintegration in the (low pH) gastric environment.

In the context of the present invention, the term "oral dosage form" is not particularly limited. As is generally understood, an oral dosage form refers to any pharmaceutical formulation that is designed to be taken by mouth and absorbed through the gastrointestinal tract. These forms include, but are not limited to, tablets, capsules, liquids, suspensions, emulsions, powders, and granules.

Before the invention is described in exemplary detail, it is to be understood that this invention is not limited to the particular component parts of the process steps of the methods described herein as such methods may vary. It is also to be understood that the terminology used herein is for purposes of describing particular embodiments only, and is not intended to be limiting. It must be noted that, as used in the specification and the appended claims, the singular forms "a," "an" and "the" include singular and/or plural referents unless the context clearly dictates otherwise. For example, the term "a" as used herein can be understood as one single entity or in the meaning of "one or more" entities. It is also to be understood that plural forms include singular and/or plural referents unless the context clearly dictates otherwise. It is moreover to be understood that, in case parameter ranges are given which are delimited by numeric values, the ranges are deemed to include these limitation values.

The present invention relates to a pharmaceutical composition for use in a method for the treatment of irritable bowel syndrome (IBS). The pharmaceutical composition comprises a probiotic comprising the bacterial strains Lactobacillus helveticus, Lacticaseibacillus rhamnosus, Lacticaseibacillus casei, Lactiplantibacillus plantarum, Lactococcus lactis, Bifidobacterium longum, Bifidobacterium breve, Bifidobacterium bifidum and Streptococcus thermophilus. Alternatively, the probiotic may consist of the bacterial strains Lactobacillus helveticus, Lacticaseibacillus rhamnosus, Lacticaseibacillus casei, Lactiplantibacillus plantarum, Lactococcus lactis, Bifidobacterium longum, Bifidobacterium bifidum, Streptococcus thermophilus and optionally Bifidobacterium breve.

While there is a lot of published scientific data about the treatment of IBS patients with probiotics and/or synbiotics, the potential effects of probiotics and/or synbiotics cannot be predicted with certainty. The influence of one probiotic cannot be extrapolated to another one from the same species or even to a different strain [31]. Certain probiotics have been found not to be effective in IBS treatment [32-38] or have been found to be only effective in stool form subtypes of IBS [39]. Furthermore, unfavorable effects in IBS patients have been found for certain probiotics [40] and synbiotics [41]. Certain synbiotics have been demonstrated to have no effects on flatulence/bloating [42]. A clinical trial investigating the effects of Balance^{®}, a synbiotic comprising 4 Lactobacilli, 3 Bifidobacteria, S. thermophilus and FOS, found no positive effects [43]. In a clinical trial studying the synbiotic Lactol^{®} in IBS-patients no improvement in constipation-related symptoms was observed [41].

In this regard, the inventors have surprisingly found that a probiotic having the specific selection of bacterial strains as defined above leads to an improvement of all major symptoms occurring in IBS patients. In particular, the pharmaceutical composition comprising the probiotic significantly reduces the severity and frequency of abdominal pain, the severity of flatulence, the dissatisfaction with bowel habit and the interference of IBS on the quality of life. Furthermore, the pharmaceutical composition comprising the probiotic is capable of improving the feeling of complete evacuation of stool. In addition, the pharmaceutical composition is generally safe and is not causing treatment related adverse events.

According to certain embodiments, the method comprises administering the pharmaceutical composition to a subject with IBS-D. Alternatively or in addition, the pharmaceutical composition may be for use in a method for the treatment of IBS-D. The inventors have surprisingly found that the treatment of IBS patients with IBS-D with the pharmaceutical composition leads to a normalization of their stool characteristics. In addition, the above-mentioned IBS-related symptoms are further reduced in IBS-D patients when treated with the pharmaceutical composition.

In some embodiments, the method comprises administering the pharmaceutical composition to a subject with IBS-C. Alternatively or in addition, the pharmaceutical composition is for use in a method for the treatment of IBS-C. The inventors have surprisingly found that the treatment of IBS patients with IBS-C with the pharmaceutical composition leads to a normalization of their stool characteristics. In addition, the above-mentioned IBS-related symptoms are further reduced in IBS-C patients when treated with the pharmaceutical composition.

In certain embodiments, the pharmaceutical composition is for use in a method for the treatment of IBS-D and IBS-C. Surprisingly, the inventors have found that the pharmaceutical composition can be successfully used both for the treatment of IBS-C or patients with IBS-C and for the treatment of IBS-D or patients with IBS-D. In particular, the treatment of IBS patients with IBS-C or IBS-D with the pharmaceutical composition leads to a normalization of their stool characteristics.

According to some embodiments, the method comprises administering the pharmaceutical composition to a subject with severe IBS. Alternatively or in addition, the pharmaceutical composition may be for use in a method for the treatment of severe IBS. The inventors have surprisingly found that the treatment of severe IBS patients with the pharmaceutical composition results in a significant improvement of symptoms associated with IBS and/or severe IBS.

According to certain embodiments, the method comprises administering the pharmaceutical composition to a subject with moderate IBS. Alternatively or in addition, the pharmaceutical composition may be for use in a method for the treatment of moderate IBS. The inventors have surprisingly found that the treatment of moderate IBS patients with the pharmaceutical composition results in a significant improvement of symptoms associated with IBS and/or moderate IBS.

In some embodiments, the pharmaceutical composition is for use in the treatment of abdominal pain associated with IBS. Alternatively or in addition, the pharmaceutical composition may be for use in the treatment of bloating and/or flatulence associated with IBS. Alternatively or in addition, the pharmaceutical composition may be for use in the treatment of stool pressure associated with IBS. Alternatively or in addition, the pharmaceutical composition may be for use in the treatment of incomplete stool evacuation associated with IBS.

According to certain embodiments, the pharmaceutical composition comprises 15% to 25%, preferably 17% to 23% and more preferably 19% to 21% Lactobacillus helveticus, 7% to 13%, preferably 8% to 12% and more preferably 9% to 11% Lacticaseibacillus rhamnosus, 3% to 7%, preferably 4% to 6% and more preferably 4,5% to 5,5% Lacticaseibacillus casei, 3% to 7%, preferably 4% to 6% and more preferably 4,5% to 5,5% Lactiplantibacillus plantarum, 15% to 25%, preferably 17% to 23% and more preferably 19% to 21% Lactococcus lactis, 18% to 32%, preferably 20% to 30% and more preferably 23% to 27% Bifidobacterium longum, 3% to 7%, preferably 4% to 6% and more preferably 4,5% to 5,5% Bifidobacterium bifidum and 7% to 13%, preferably 8% to 12% and more preferably 9% to 11% Streptococcus thermophilus. The percentage "%" refers to "% of total CFU (colony forming units) contained in the probiotic". Alternatively or in addition, the percentage "%" refers to "% of total CFU contained in the pharmaceutical composition". Preferably, the percentage "%" refers to "% of total CFU contained in the pharmaceutical composition". In these embodiments, the above-mentioned advantages and effects of the invention are further improved.

According to certain embodiments, the pharmaceutical composition consists of 15% to 25%, preferably 17% to 23% and more preferably 19% to 21% Lactobacillus helveticus, 7% to 13%, preferably 8% to 12% and more preferably 9% to 11% Lacticaseibacillus rhamnosus, 3% to 7%, preferably 4% to 6% and more preferably 4,5% to 5,5% Lacticaseibacillus casei, 3% to 7%, preferably 4% to 6% and more preferably 4,5% to 5,5% Lactiplantibacillus plantarum, 15% to 25%, preferably 17% to 23% and more preferably 19% to 21% Lactococcus lactis, 18% to 32%, preferably 20% to 30% and more preferably 23% to 27% Bifidobacterium longum, 3% to 7%, preferably 4% to 6% and more preferably 4,5% to 5,5% Bifidobacterium bifidum and 7% to 13%, preferably 8% to 12% and more preferably 9% to 11% Streptococcus thermophilus, wherein the sum of the percentages equals 100%. The percentage "%" refers to "% of total CFU (colony forming units) contained in the probiotic". Alternatively or in addition, the percentage "%" refers to "% of total CFU contained in the pharmaceutical composition". Preferably, the percentage "%" refers to "% of total CFU contained in the pharmaceutical composition". In these embodiments, the above-mentioned advantages and effects of the invention are further improved.

According to certain embodiments, the pharmaceutical composition comprises 15% to 25%, preferably 17% to 23% and more preferably 19% to 21% Lactobacillus helveticus, 7% to 13%, preferably 8% to 12% and more preferably 9% to 11% Lacticaseibacillus rhamnosus, 3% to 7%, preferably 4% to 6% and more preferably 4,5% to 5,5% Lacticaseibacillus casei, 3% to 7%, preferably 4% to 6% and more preferably 4,5% to 5,5% Lactiplantibacillus plantarum, 15% to 25%, preferably 17% to 23% and more preferably 19% to 21% Lactococcus lactis, 11% to 19%, preferably 12% to 18% and more preferably 14% to 16% Bifidobacterium longum, 7% to 13%, preferably 8% to 12% and more preferably 9% to 11% Bifidobacterium breve, 3% to 7%, preferably 4% to 6% and more preferably 4,5% to 5,5% Bifidobacterium bifidum and 7% to 13%, preferably 8% to 12% and more preferably 9% to 11% Streptococcus thermophilus. The percentage "%" refers to "% of total CFU (colony forming units) contained in the probiotic". Alternatively or in addition, the percentage "%" refers to "% of total CFU contained in the pharmaceutical composition". Preferably, the percentage "%" refers to "% of total CFU contained in the pharmaceutical composition". In these embodiments, the above-mentioned advantages and effects of the invention are further improved.

In some embodiments, the pharmaceutical composition consists of 15% to 25%, preferably 17% to 23% and more preferably 19% to 21% Lactobacillus helveticus, 7% to 13%, preferably 8% to 12% and more preferably 9% to 11% Lacticaseibacillus rhamnosus, 3% to 7%, preferably 4% to 6% and more preferably 4,5% to 5,5% Lacticaseibacillus casei, 3% to 7%, preferably 4% to 6% and more preferably 4,5% to 5,5% Lactiplantibacillus plantarum, 15% to 25%, preferably 17% to 23% and more preferably 19% to 21% Lactococcus lactis, 11% to 19%, preferably 12% to 18% and more preferably 14% to 16% Bifidobacterium longum, 7% to 13%, preferably 8% to 12% and more preferably 9% to 11% Bifidobacterium breve, 3% to 7%, preferably 4% to 6% and more preferably 4,5% to 5,5% Bifidobacterium bifidum and 7% to 13%, preferably 8% to 12% and more preferably 9% to 11% Streptococcus thermophilus, wherein the sum of the percentages equals 100%. The percentage "%" refers to "% of total CFU (colony forming units) contained in the probiotic". Alternatively or in addition, the percentage "%" refers to "% of total CFU contained in the pharmaceutical composition". Preferably, the percentage "%" refers to "% of total CFU contained in the pharmaceutical composition". In these embodiments, the above-mentioned advantages and effects of the invention are further improved.

According to some embodiments, the pharmaceutical composition comprises or consists of 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactobacillus helveticus, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Lacticaseibacillus rhamnosus, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lacticaseibacillus casei, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lactiplantibacillus plantarum, 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactococcus lactis, 8.75 × 10⁸ to 13.75 × 10⁸ CFU, preferably 9.75 × 10⁸ to 12.75 × 10⁸ CFU and more preferably 10.50 × 10⁸ to 12.00 × 10⁸ CFU Bifidobacterium longum, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Bifidobacterium bifidum and 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Streptococcus thermophilus. In these embodiments, the above-mentioned advantages and effects of the invention are further improved.

According to some embodiments, the pharmaceutical composition comprises 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactobacillus helveticus, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Lacticaseibacillus rhamnosus, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lacticaseibacillus casei, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lactiplantibacillus plantarum, 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactococcus lactis, 5.25 × 10⁸ to 8.25 × 10⁸ CFU, preferably 5.75 × 10⁸ to 7.75 × 10⁸ CFU and more preferably 6.25 × 10⁸ to 7.25 × 10⁸ CFU Bifidobacterium longum, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Bifidobacterium breve, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Bifidobacterium bifidum and 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Streptococcus thermophilus. In these embodiments, the above-mentioned advantages and effects of the invention are further improved.

In certain embodiments, the pharmaceutical composition consists of 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactobacillus helveticus, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Lacticaseibacillus rhamnosus, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lacticaseibacillus casei, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Lactiplantibacillus plantarum, 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU Lactococcus lactis, 5.25 × 10⁸ to 8.25 × 10⁸ CFU, preferably 5.75 × 10⁸ to 7.75 × 10⁸ CFU and more preferably 6.25 × 10⁸ to 7.25 × 10⁸ CFU Bifidobacterium longum, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Bifidobacterium breve, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU Bifidobacterium bifidum and 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU Streptococcus thermophilus. In these embodiments, the above-mentioned advantages and effects of the invention are further improved.

According to certain embodiments, the lactobacillus helveticus is lactobacillus helveticus SP 27. Alternatively or in addition, the lacticaseibacillus rhamnosus may be lacticaseibacillus rhamnosus Lr-32. Alternatively or in addition, the lacticaseibacillus casei may be lacticaseibacillus casei Lc-11. Alternatively or in addition, the lactiplantibacillus plantarum may be lactiplantibacillus plantarum Lp-115. Alternatively or in addition, the lactococcus lactis may be lactococcus lactis LI-23. Alternatively or in addition, the bifidobacterium longum may be bifidobacterium longum BI-05 and/or Bifidobacterium longum ES1. Alternatively or in addition, the bifidobacterium longum may be bifidobacterium longum BI-05. Alternatively or in addition, the bifidobacterium longum may be bifidobacterium longum BI-05 and Bifidobacterium longum ES1. Alternatively or in addition, the bifidobacterium breve may be bifidobacterium breve Bb-03. Alternatively or in addition, the bifidobacterium breve may be Bifidobacterium longum ES1. Alternatively or in addition, the bifidobacterium bifidum may be bifidobacterium bifidum Bb-02. Alternatively or in addition, the streptococcus thermophilus may be streptococcus thermophilus St-21. In these embodiments, the above-mentioned advantages and effects of the invention are further improved. In the context of the present invention, the term "bifidobacterium breve" may be understood to include Bifidobacterium longum ES1.

In some embodiments, the lactobacillus helveticus is lactobacillus helveticus SP 27, the lacticaseibacillus rhamnosus is lacticaseibacillus rhamnosus Lr-32, the lacticaseibacillus casei is lacticaseibacillus casei Lc-11, the lactiplantibacillus plantarum is lactiplantibacillus plantarum Lp-115, the lactococcus lactis is lactococcus lactis LI-23, the bifidobacterium longum is bifidobacterium longum BI-05, the bifidobacterium breve is bifidobacterium breve Bb-03, the bifidobacterium bifidum is bifidobacterium bifidum Bb-02, and the streptococcus thermophilus is streptococcus thermophilus St-21. In these embodiments, the above-mentioned advantages and effects of the invention are further improved.

In some embodiments, the lactobacillus helveticus is lactobacillus helveticus SP 27, the lacticaseibacillus rhamnosus is lacticaseibacillus rhamnosus Lr-32, the lacticaseibacillus casei is lacticaseibacillus casei Lc-11, the lactiplantibacillus plantarum is lactiplantibacillus plantarum Lp-115, the lactococcus lactis is lactococcus lactis LI-23, the bifidobacterium longum is bifidobacterium longum BI-05 and bifidobacterium longum ES1, the bifidobacterium bifidum is bifidobacterium bifidum Bb-02, and the streptococcus thermophilus is streptococcus thermophilus St-21. In these embodiments, the above-mentioned advantages and effects of the invention are further improved.

In some embodiments, the lactobacillus helveticus is lactobacillus helveticus SP 27, the lacticaseibacillus rhamnosus is lacticaseibacillus rhamnosus Lr-32, the lacticaseibacillus casei is lacticaseibacillus casei Lc-11, the lactiplantibacillus plantarum is lactiplantibacillus plantarum Lp-115, the lactococcus lactis is lactococcus lactis LI-23, the bifidobacterium longum is bifidobacterium longum BI-05, the bifidobacterium breve is bifidobacterium longum ES1, the bifidobacterium bifidum is bifidobacterium bifidum Bb-02, and the streptococcus thermophilus is streptococcus thermophilus St-21. In these embodiments, the above-mentioned advantages and effects of the invention are further improved.

According to some embodiments, the pharmaceutical composition comprises 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU lactobacillus helveticus SP 27, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU lacticaseibacillus rhamnosus Lr-32, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU lacticaseibacillus casei Lc-11, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU lactiplantibacillus plantarum Lp-115, 7.00 × 10⁸ to 11.00 × 10⁸ CFU, preferably 8.00 × 10⁸ to 10.00 × 10⁸ CFU and more preferably 8.50 × 10⁸ to 9.50 × 10⁸ CFU lactococcus lactis Ll-23, 5.25 × 10⁸ to 8.25 × 10⁸ CFU, preferably 5.75 × 10⁸ to 7.75 × 10⁸ CFU and more preferably 6.25 × 10⁸ to 7.25 × 10⁸ CFU bifidobacterium longum BI-05, 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU bifidobacterium longum ES1, 1.75 × 10⁸ to 2.75 × 10⁸ CFU, preferably 2.00 × 10⁸ to 2.50 × 10⁸ CFU and more preferably 2.10 × 10⁸ to 2.40 × 10⁸ CFU bifidobacterium bifidum Bb-02and 3.50 × 10⁸ to 5.50 × 10⁸ CFU, preferably 4.00 × 10⁸ to 5.00 × 10⁸ CFU and more preferably 4.25 × 10⁸ to 4.75 × 10⁸ CFU streptococcus thermophilus St-21. In these embodiments, the above-mentioned advantages and effects of the invention are further improved.

In certain embodiments, the pharmaceutical composition is a synbiotic. According to some embodiments, the pharmaceutical composition further comprises at least one prebiotic. In certain embodiments, the at least one prebiotic is selected from the group consisting of fructooligosaccharides (FOS), galactooligosaccharides, inulin and combinations thereof. The at least one prebiotic may be fructooligosaccharides (FOS). Alternatively or in addition, the pharmaceutical composition may comprise 20 mg to 100 mg, preferably 40 mg to 80 mg and more preferably 50 mg to 70 mg of the at least one prebiotic. Alternatively or in addition, the pharmaceutical composition may comprise 20 mg to 100 mg, preferably 40 mg to 80 mg and more preferably 50 mg to 70 mg of fructooligosaccharides. In these embodiments, the above-mentioned advantages and effects of the invention are further improved.

According to certain embodiments, the pharmaceutical composition is in oral dosage form. In some embodiments, the oral dosage form is selected from the group consisting of tablets, capsules, liquids, suspensions, emulsions, powders, and granules. According to some embodiments, the probiotic is contained in a capsule. In certain embodiments, the probiotic and the prebiotic are contained in a capsule.

Pharmaceutical capsules are typically made from one of two types of materials: gelatin or a vegetarian substitute. Each material is chosen based on its ability to dissolve at the right location in the digestive tract, ensuring the medicine within is released where it can be most effective.

Gelatin Capsules: Traditionally, capsules have been made from gelatin, which is a clear, tasteless substance derived from collagen obtained from various animal by-products. Gelatin capsules are popular because they are easy to digest and start to dissolve as soon as they reach the stomach, releasing the medication inside quickly.

Vegetarian Capsules: As an alternative to gelatin, vegetarian capsules are made from cellulose, the main substance in plant cells, or other plant-derived materials like hypromellose (hydroxypropyl methylcellulose or HPMC). Vegetarian capsules offer the same benefits as gelatin capsules, such as dissolving at controlled rates and being easy to digest, but are made from non-animal sources.

In some embodiments, the capsule comprises an enteric coating. The enteric coating may comprise gellan gum (E418), magnesium salts of fatty acids (E470b), shellac (E904), ascorbic acid (E300), alginic acid (E400), triethyl citrate (E1505), locust bean gum (E410) and/or olive oil. In these embodiments, the above-mentioned advantages and effects of the invention are further improved.

In some embodiments, Vivatlac^{®} Synbiotikum of Vivatrex^{®} GmbH can be used as pharmaceutical composition in accordance with the present invention.

According to certain embodiments, the method comprises orally administering the pharmaceutical composition to a subject. In some embodiments, the method comprises administering the pharmaceutical composition to a subject once daily. In certain embodiments, the subject is an IBS patient. Alternatively or in addition, the subject may be a patient suffering from IBS-D or IBS-C. Alternatively or in addition, the subject may be a patient suffering from severe IBS or moderate IBS. In certain embodiments, the subject and/or patient is human. In these embodiments, the above-mentioned advantages and effects of the invention are further improved.

The above embodiments can be combined arbitrarily, if appropriate. Further possible embodiments and implementations of the invention comprise also combinations of features not explicitly mentioned in the foregoing or in the following with regard to the Examples of the invention. Particularly, a person skilled in the art will also add individual aspects as improvements or additions to the respective basic form of the invention.

### Examples

The present invention will now be described in detail with reference to several examples thereof. However, these examples are illustrative and do not limit the scope of the invention.

### Example 1: Exemplary pharmaceutical composition

Table 1 shows an exemplary composition of a probiotic. The mixture of nine probiotic bacteria strains are contained in a capsule. Each capsule contains a total of 45.0 x 10⁸ colony forming units (CFU). Individual probiotic strains contribute from 5 to 20% of the total amount of probiotic bacteria with their amounts of CFU per capsule ranging from 2.25 x 10⁸ to 9.00 x 10⁸. In addition, each capsule contains 63 mg of fructooligosaccharides (FOS) as a prebiotic component. The capsule is a gelatine-free hydroxypropyl-methylcellulose capsule which is enteric-coated to protect the probiotic bacteria against destruction by stomach acid.

**Table 1: Exemplary composition of a probiotic contained in a pharmaceutical composition**

| **Probiotic Strain** | **Colony-Forming Units (CFU) per capsule'** | **% of total CFU** |
|---|---|---|
| *Lactobacillus helveticus SP 27* | 9.00 x 10⁸ | 20 |
| *Lacticaseibacillus rhamnosus Lr-32* | 4.50 x 10⁸ | 10 |
| *Lacticaseibacillus casei Lc-11* | 2.25 x 10⁸ | 5 |
| *Lactiplantibacillus plantarum Lp-115* | 2.25 x 10⁸ | 5 |
| *Lactococcus lactis Ll-23* | 9.00 x 10⁸ | 20 |
| *Bifidobacterium longum Bl-05* | 6.75 x 10⁸ | 15 |
| *Bifidobacterium breve Bb-03* | 4.50 x 10⁸ | 10 |
| *Bifidobacterium bifidum Bb-02* | 2.25 x 10⁸ | 5 |
| *Streptococcus thermophilus St-21* | 4.50 x 10⁸ | 10 |
| **Total CFU** | **45.00 x 10⁸** | **100** |

### Example 2: Clinical evaluation of the pharmaceutical composition according to Example 1

### Material and method

The study design was a multi-center, randomized, double-blind, placebo-controlled clinical trial. A total of 8 physicians enrolled patients at the Family doctor's clinic, in 62-820 Stawiszyn, West-Poland and at the family doctor's outpatient clinic Panacea, in 27-230 Krynki, East-Poland. Each individual patient was cared for from the start to the end of the trial by one physician only. The first patient was enrolled in February 2023 and the treatment of the last patient was finished in November 2023. Neither patients nor physicians were incentivized for participating in the trial. The study protocol was approved by the Ethics Committee of the Calisia University (project identification code 1/2023) and the trial performed was conducted in accordance with the Declaration of Helsinki.

### Patients

The study recruited female and male patients aged 18 to 65 years diagnosed for irritable bowel syndrome according to the IBS questionnaire for health care providers developed by the World Gastroenterology Organization [44] and having an IBS severity assessed by using the IBS-SSS of ≥ 175 points representing moderate to severe cases of IBS [8].

Exclusion criteria included: use of products containing probiotic bacteria or treatment with antibiotics within the last three months, concurrent severe illness (malignancies, uncontrolled hypertension or diabetes, hepatic, renal or cardiac dysfunctions, serious neurological disorders, psychosis, respiratory disorders such as asthma or chronic obstructive pulmonary disease, hyper- or hypothyroidism), chronic bowel disorders other than IBS, including inflammatory bowel disease, gastroenteritis, stomach and duodenal cancer, celiac disease, pregnancy or lactation, diagnosed lactose intolerance, use of motility drugs or dietary fiber supplements within 2 weeks before the study start, taking anti-coagulant medication, participation in another clinical trial within the last three months. Patients who received antibiotics during the study were excluded.

### Pharmaceutical composition and placebo preparations

The pharmaceutical composition is commercially available (Vivatlac^{®} Synbiotikum, Vivatrex^{®} GmbH, Groiner Kirchweg 68, 46459 Rees, Germany) and was purchased for the trial. Placebo capsules identical in appearance, size and weight containing maize starch were produced by Solger SP (Kamienica 40, 62-530 Kamienica, Polen). Placebo and verum were blinded by packaging in white plastic bottles individually labeled according to the randomization list created for the two treatment arms of the trial. Each bottle contained 84 capsules covering the 12 weeks treatment period for each trial participant.

### Design of study

The study design is summarized in Figure 1. Physicians reported all result of their examinations in a printed Physicians' Data Log-Book. During an initial visit (visit 0) patients underwent an in-depth physical examination, diagnosis for IBS using the World Gastroenterology questionnaire for HCP, determination of IBS severity using the IBS-SSS, determination of the type of IBS using the Bristol Stool Form Scale, check of inclusion and exclusion criteria. During this visit the patients were asked for their consent to participate in the study. Patients were provided with a Patients' Diary and instructed how to use it for reporting of symptoms and adverse events.

A four weeks screening phase was used to evaluate patients' IBS and their capability to report IBS symptoms using a patient diary (Fig. 1). At the end of the screening phase participants were randomly assigned (1:1) to treatment with the pharmaceutical composition (one capsule per day), also hereinafter referred to as the "multi-strain synbiotic", or placebo for 12 weeks of treatment.

### Sample size calculation

Assuming a significance level of 5%, a power of 90%, a responder rate of 25% in the placebo group and of 50% in the multi-strain synbiotic group and a dropout rate of 25%, a required sample size per treatment arm of 100 patients was calculated by using the Sample Size Calculator of Sealed Envelope Ltd. (London, UK).

### Randomization

Patients were randomly assigned to the two treatment-groups. A two-treatment equal allocation (1:1) randomization scheme was generated by using the Simple Randomiser of Sealed envelope Ltd. (London, UK). Treatment was allocated to patients by physician using the randomized scheme based on entry into the study.

### IBS-Type categorization

Patients were categorized as IBS-D patients when they reported during the 4 weeks screening phase only Bristol stool scores of 5, 6 or 7. If patients reported only scores of 1 or 2 they were categorized as IBS-C patients. Patients reporting during screening phase at least one 5, 6 or 7 score and at least one 1 or 2 score were categorized as IBS-M types. Patients who reported during the first 4 weeks scores of 3 or 4 or patients without the full set of 4 scores were categorized as unspecified IBS (IBS-U) patients.

### Primary Endpoints

Primary efficacy endpoints defined for the trial were: (i) assessment of the severity of IBS symptoms using the IBS Severity of Symptoms Scale (IBS-SSS), (ii) assessment of improvement or worsening of IBS global symptoms using the Global Improvement Scale (IBS-GIS).

IBS-SSS is a five-question survey evaluating for the last 10 days: (1) the severity of abdominal pain (IBS-SSS1), (2) the frequency of abdominal pain (IBS-SSS2), (3) the severity of abdominal flatulence (IBS-SSS3), (4) the dissatisfaction with bowel habit (IBS-SSS4) and (5) the interference with quality of life (IBS-SSS5) [8]. Patients responded to each question on a 100-point visual analogue scale. Each of the five questions generated a maximum score of 100 points, and total scores could range from 0-500 with higher scores indicating severe symptoms or higher burden for patients. When registering the trial we, as done by many others, assumed that patients with decreases of ≥ 50 points on the IBS-SSS at visit 4 compared to the value at visits 0 could be considered to be responders.

With the IBS-GIS IBS symptoms are assessed by using a patient defined 7-point Likert scale ranging from symptoms substantially worse (1 point) to substantially improved (7 points) [45]. Patients answered the question "Have you felt any change in the severity of your symptoms over the past 7 days compared to how you felt before the medication was taken?" Answers were recorded based on the 7-point scale: 1 point - "I feel that the symptoms have worsened significantly"; 2 points - "I feel that the symptoms have moderately worsened "; 3 points - "I feel that the symptoms have slightly worsened"; 4 points - "I feel no change"; 5 points - "I feel a slight improvement"; 6 points - "I feel a moderate improvement"; 7 points - "I feel a significant improvement". IBS-GIS score indicated an improvement if score was >4, worsening if it was <4 and no change if it was 4. Patients for which a change on the IBS-GIS of 3 points was reported were considered as responder.

### Secondary Endpoints

Secondary efficacy endpoints included the IBS-Adequate Relief (IBS-AR) which is a dichotomous single item that asked trial participants "Over the past week have you had adequate relief of your IBS-symptoms?" [46]. The answer was "yes" or "no". IBS-AR scores were assessed during visits 1 to 4.

Other secondary efficacy endpoints were (i) type of stool assessed using the Bristol Stool Form Scale, (ii) the severity of abdominal pain, (iii) the severity of flatulence, (iv) stool pressure, (v) the feeling of incomplete evacuation of stool and (vi) adverse events. All these secondary endpoint information was collected by the use of a patient diary into which patients reported on a weekly basis during the 4 weeks before the treatment start and the 12 weeks of treatment. During the visit 1 the quality of the patients' reporting (e.g., all questions answered) were checked and in case it was not satisfying patients were retrained how to use the diary.

The stool types were assessed by using the Bristol Stool Form Scale (visualized in the patient diary) that classifies feces into seven groups: types 1-2 indicate constipation; types 3-4 are normal stools and types 5-7 indicate diarrhea.

IBS-symptoms except for a feeling of incomplete bowel movement were assessed using a patient-defined 5-point Likert scale: 0 points indicating no symptoms and 1-4 indicating the severity of symptoms with higher scores indicating worse symptoms. The feeling of incomplete bowel movement was assessed using a 2-point scale: 0 - no such feeling and 1 - there is an incomplete bowel movement. Adverse events were evaluated on a two item scale (yes or no) and a field for free text to describe the nature of any adverse event.

### Results

### Patient flow, study progress and baseline characteristics of patient groups

Figure 2 shows the flow of patients through the study. During the recruitment period, a total of 745 patients were assessed for eligibility to participate in the study. Of these, 417 were excluded because of no IBS, 89 because they refused to participate in the trial, and 37 because they met at least one of the exclusion criteria. The remaining 202 patients were randomly and equally allocated to the placebo arm (n=101) or the multi-strain synbiotic arm (n=101). One patient of the placebo group was lost in follow-up. Data from 100 patients of the placebo group and 101 patients of the multi-strain synbiotic group were analyzed.

Patients baseline characteristics are shown in Table 2. The ratio between female and male patients was 1.45. Patients with moderate IBS at the beginning of the study represented 54.7% of study participants. IBS with diarrhea was diagnosed in 72.6% and IBS with constipation in 22.9% of patients. There were no statistically significant differences between the placebo and the multi-strain synbiotic groups in weight, height, BMI, age, gender, share of IBS-types and IBS severity.

**Table 2: Baseline characteristic of patients at the start of the clinical trial (visit 0)**

| **Characteristics (Visit 0)** | **Unit** | **Placebo** | | **Pharmaceutical composition** | | **All** | |
|---|---|---|---|---|---|---|---|
| | | avg | S.D. | avg | S.D. | avg | S.D. |
| Number of pts | # of pts | 100 | | 101 | | 201 | |
| Weight | kg | 72.1 | 12.3 | 70.1 | 12.3 | 71.1 | 12.3 |
| Height | m | 172.8 | 9.5 | 171.2 | 9.3 | 172.0 | 9.4 |
| BMI | kg/m² | 24.2 | 2.1 | 23.9 | 2.1 | 23.8 | 2.1 |
| Age | years | 40.8 | 10.7 | 41.9 | 9.5 | 41.3 | 10.1 |
| Male | # of pts | 45 | | 37 | | 82 | |
| Male | % of T. | 54.0 | | 36.6 | | 40.8 | |
| Female | # of pts | 55 | | 54 | | 119 | |
| Female | % of T. | 55.0 | | 63.4 | | 59.2 | |
| IBS-D | # of pts | 73 | | 73 | | 146 | |
| IBS-D | % of T. | 73.0 | | 72.3 | | 72.6 | |
| IBS-C | # of pts | 20 | | 26 | | 46 | |
| IBS-C | % of T. | 20.0 | | 25.7 | | 22.9 | |
| IBS-M | # of pts | 2 | | 1 | | 3 | |
| IBS-M | % of T. | 2.0 | | 1.0 | | 1.5 | |
| IBS-U | # of pts | 5 | | 1 | | 6 | |
| IBS-U | % of T. | 5.0 | | 1.0 | | 3.0 | |
| Moderate IBS | # of pts | 57 | | 53 | | 110 | |
| Moderate IBS | % of T. | 57.0 | | 52,5 | | 54.7 | |
| Severe IBS | # of pts | 43 | | 48 | | 91 | |
| Severe IBS | % of T. | 43.0 | | 47.5 | | 45.3 | |
| Bristol Score IBS-D | Score | 6.4 | 0.5 | 6.4 | 0.5 | 6.4 | 0.5 |
| Bristol Score IBS-C | Score | 1.6 | 0.5 | 1.6 | 0.5 | 1.6 | 0.5 |
| Score WGO | Score | 25.0 | 1.2 | 24.8 | 1.6 | 24.9 | 1.4 |
| IBS-SSS | Score | 295.1 | 23.9 | 300.5 | 25.7 | 297.8 | 24.8 |
| IBS-SSS1 | Score | 59.1 | 6.3 | 59.9 | 6.7 | 59.5 | 6.2 |
| IBS-SSS2 | Score | 58.3 | 5.8 | 58.4 | 6.0 | 58.3 | 5.9 |
| IBS-SSS3 | Score | 58.7 | 5.9 | 60.7 | 6.1 | 59.7 | 6.1 |
| IBS-SSS4 | Score | 59.3 | 6.1 | 59.8 | 7.8 | 59.6 | 7.0 |
| IBS-SSS5 | Score | 59.8 | 6.5 | 61.8 | 6.1 | 60.8 | 6.3 |

### Assessment of severity of symptoms with IBS-SSS (primary endpoint)

Reduction of IBS-symptoms determined using the IBS-SSS was one of the two primary endpoints defined for the trial (Figure 3). The mean values of the total IBS-SSS at the beginning of the run-in phase (visit 0) were similar in both groups: 295.1 ± 23.9 and 300.5 ± 25.7 in the placebo and multi-strain synbiotic groups, respectively. At the end of the run-in phase these values had hardly changed 289.8 ± 25.0 and 290.4 ± 26.2 in the placebo and multi-strain synbiotic groups, respectively. A decrease in IBS severity compared to the baseline values (visit 0 and visit 1) was observed for the multi-strain synbiotic group after 4, 8 and 12 weeks of treatment. The decreases observed after 8 and after 12 weeks of treatment were statistically significant compared to the baseline values determined for this patient group and compared to the values determined after 8 and 12 weeks for the placebo group. After 4 weeks the IBS severity in the multi-strain synbiotic group was reduced, however the effect failed to reach statistically significance. Analyses of the domain specific scores (IBS-SSS1 to IBS-SSS5) revealed that the multi-strain synbiotic resulted in statistically significant decreases on all sub-scales after 8 and 12 weeks of treatment (Figures 4 to 8).

Using a reduction of ≥ 50 as cut-off for calculation of responders at the end of the 12 weeks treatment period versus the baseline value (visit 0) resulted in a responder rate of 14% for the placebo group and of 98% for the multi-strain synbiotic group.

### Effects of treatments on IBS-SSS sub-scale measures

Table 3 shows that the pharmaceutical composition (multi-strain synbiotic) improves IBS symptoms assessed by the sub-scales of IBS-SSS to similar extents.

**Table 3: Relative changes (V4/V0-1) of IBS symptoms assessed by the sub-scales of IBS-SSS for the group of patients treated with placebo and the group treated with the pharmaceutical composition**

| IBS symptom | Group treated placebo (relative change V4/V0-1) | Group treated with pharmaceutical composition (relative change V4/V0-1) |
|---|---|---|
| Severity of abdominal pain | -4.2% | -48.3% |
| Frequency of abdominal pain | -4.0% | -48.7% |
| Severity of flatulence | -2.5% | -50.6% |
| Dissatisfaction with bowel habit | -4.7% | -52.1% |
| Interference with Quality of Life | -3.9% | -55.7% |

### Change of IBS severity assessed by IBS-SSS

As shown in Figure 10, the treatment of IBS patients with the multi-strain synbiotic reduced the severity of IBS symptoms. No such effect was observed in patients receiving placebo (see Figure 9). After 12 weeks of treatment with the multi-strain synbiotics nearly 80 percent of patients were assessed to have only mild IBS-symptoms. Figures 11-14 show that the pharmaceutical composition works particularly well for patients having moderate or severe IBS. In particular, Figure 11 shows that the group of patients with severe IBS showed only mild to moderate IBS symptoms after twelve weeks of treatment with the pharmaceutical composition.

### Assessment of global improvement with IBS-GIS (primary endpoint)

Global improvement of IBS assessed by the use of the IBS-GIS was defined as the second primary endpoint of the trial. Treatment effects on measures using the IBS-GIS are shown in Figure 15. At the end of the run in-phase (V1) neither for patients from the placebo nor for patients from the multi-strain synbiotic group a change of symptoms was reported using the IBS-GIS. For the placebo group no change of the IBS-GIS was observed during the treatment phase of the trial (V2 to V4). For the multi-strain synbiotic group the IBS-GIS values steadily increase with progression of treatment duration. The IBS-GIS value determined for the multi-strain synbiotic group at the end of the trial (V4) was 6.5 ± 0.9, while for the placebo group it was 4.0 ± 0.5. At the end of the treatment period (V4) 89.1% of patients of the multi-strain synbiotic group experienced a change on the IBS-GIS of at least 2 points. A change of 3 points on the IBS-GIS was observed for 52.5% of patients of the multi-strain synbiotic group.

### Assessment of adequate relief with IBS-AR Scale (secondary endpoint)

Measurement of adequate relief using the IBS-AR scale was defined as on the secondary outcomes of the trial. As shown in Figure 16, no patient in the placebo group experienced adequate relief. In the multi-strain synbiotic group, 70% of patients achieved adequate relief after 12 weeks of treatment. After 4 and 8 weeks of treatment adequate relief was achieved by 1% and 14% of patients in the multi-strain synbiotic group, respectively.

### Patients' assessment of stool consistency with Bristol Stool Scale (secondary endpoint)

Patients reported on a weekly basis the consistency of their stool using the Bristol Stool Form Scale. The four assessments of stool consistency during the run in-phase (no treatment) were used to categorize patients according to their stool consistency into IBS-D, IBS-C, IBS-M and IBS-U subgroups (Table 2). There was a total of 146 patients with IBS-D who were randomly assigned to the placebo group (73 patients) and the multi-strain synbiotic group (73 patients). Of the 46 patients diagnosed as IBS-C patients 20 patients were randomly allocated to the placebo group and 26 patients to the multi-strain synbiotic group. Changes of stool consistency were separately analyzed for the IBS-D (Figure 17) and IBS-C patient group (Figure 18).

### Patients' assessment of stool consistency with Bristol Stool Form Scale for IBS-D patients (secondary endpoint)

As shown in Figure 17, during the run-in phase of the trial IBS-D patients had a Bristol Stool Form Scale value of 6.4 ± 0.5. There was no difference between the Bristol Stool Form Scale baselevel values of the placebo and the multi-strain synbiotic group. Over the course of the treatment, no significant change of stool consistency was observed in the placebo group. Treatment with the multi-strain synbiotic resulted in a gradual decrease of the Bristol Stool Scale value. At the end of the treatment the average Bristol Stool Scale value of patients in the multi-strain synbiotic group was 4.0 ± 0.7.

### Patients' assessment of stool consistency with Bristol Stool Form Scale for IBS-C patients (secondary endpoint)

During the run-in phase the Bristol Stool Form Scale value of IBS-C patients was 1.6 ± 0.5 (Figure 18). There was no difference between the Bristol Stool Form Scale baselevel values of the placebo and the multi-strain synbiotic group. Over the course of the treatment, no significant change of stool consistency was observed for IBS-C patients in the placebo group. Treatment of IBS-C patients with the multi-strain synbiotic resulted in a gradual increase of the Bristol Stool Form Scale value. At the end of the treatment the average Bristol Stool Form Scale value of patients in the multi-strain synbiotic group was 3.2 ± 0.6.

### Patients' assessment of severity of abdominal pain (secondary endpoint)

Patients reported on a weekly the severity of abdominal pain using a 5 point Likert scale (Figure 19). Pain severity reported by patients of the placebo group (n=100) and the multi-strain synbiotic group (n=101) during the 4 weeks (weeks 1 to 4) of the run-in phase (no treatment) were similar. Treatment with placebo resulted in no change of the pain level compared to that reported for the run-in phase. In contrast, patients receiving the multi-strain synbiotic group reported a gradual decrease of abdominal pain.

### Patients' assessment of severity of bloating (secondary endpoint)

Patients reported on a weekly basis the severity of bloating using a 5 point Likert scale (Figure 20). Bloating severity reported by patients of the placebo group (n=100) and the multi-strain synbiotic group (n=101) during the 4 weeks (weeks 1 to 4) of the run-in phase (no treatment) were similar. Treatment with placebo resulted in no change of the bloating severity level compared to that reported for the run-in phase. In contrast, patients receiving the multi-strain synbiotic group reported a gradual decrease of severity of bloating.

### Patients' assessment of stool pressure (secondary endpoint)

Patients assessed their stool pressure on a weekly basis using a 5 point Likert scale (Figure 21). Stool pressure reported by patients of the placebo group (n=100) and the multi-strain synbiotic group (n=101) during the 4 weeks (weeks 1 to 4) of the run-in phase (no treatment) were similar. Treatment with placebo resulted in no change of the stool pressure compared to that reported for the run-in phase. In contrast, patients receiving the multi-strain synbiotic group reported a gradual decrease of stool pressure.

### Patients' assessment of feeling of incomplete evacuation of stool (secondary endpoint)

Patients reported on a weekly basis if evacuation of stool was incomplete on a dichotomous scale (yes or no) (Figure 22). About 10% of patients from the placebo group reported that they achieved complete stool evacuation. The percentage of patients of the multi-strain synbiotic group who reported complete stool evacuation started to gradually increase after 6 weeks of treatment. At the end of the 12 weeks treatment period some 90% of the patients taking the multi-strain synbiotic reported a feeling of complete stool evacuation.

### References

[1] Ford AC, Sperber AD, Corsetti M, Camilleri M. Irritable bowel syndrome. Lancet. 2020 Nov 21;396(10263):1675-1688. doi: 10.1016/S0140-6736(20)31548-8. Epub 2020 Oct 10. PMID: 33049223.
[2] Black CJ, Ford AC. Global burden of irritable bowel syndrome: trends, predictions and risk factors. Nat Rev Gastroenterol Hepatol. 2020 Aug;17(8):473-486. doi: 10.1038/s41575-020-0286-8. Epub 2020 Apr 15. PMID: 32296140.
[3] Camilleri M. Diagnosis and Treatment of Irritable Bowel Syndrome: A Review. JAMA. 2021 Mar 2;325(9):865-877. doi: 10.1001/jama.2020.22532. Erratum in: JAMA. 2021 Apr 20;325(15):1568. PMID: 33651094.
[4] Drossman DA, Chang L, Schneck S, Blackman C, Norton WF, Norton NJ. A focus group assessment of patient perspectives on irritable bowel syndrome and illness severity. Dig Dis Sci. 2009 Jul;54(7):1532-41. doi: 10.1007/s10620-009-0792-6. Epub 2009 Apr 1. PMID: 19337833.
[5] Tornkvist NT, Aziz I, Whitehead WE, Sperber AD, Palsson OS, Hreinsson JP, Simrén M, Törnblom H. Health care utilization of individuals with Rome IV irritable bowel syndrome in the general population. United European Gastroenterol J. 2021 Dec;9(10):1178-1188. doi: 10.1002/ueg2.12153. Epub 2021 Oct 1. PMID: 34599559; PMCID: PMC8672084.
[6] Shah ED, Salwen-Deremer JK, Gibson PR, Muir JG, Eswaran S, Chey WD. Comparing Costs and Outcomes of Treatments for Irritable Bowel Syndrome With Diarrhea: Cost-Benefit Analysis. Clin Gastroenterol Hepatol. 2022 Jan;20(1):136-144.e31. doi: 10.1016/j.cgh.2020.09.043. Epub 2020 Oct 1. PMID: 33010413.
[7] Sood R, Law GR, Ford AC. Diagnosis of IBS: symptoms, symptom-based criteria, biomarkers or 'psychomarkers'? Nat Rev Gastroenterol Hepatol. 2014 Nov;11(11):683-91. doi: 10.1038/nrgastro.2014.127. Epub 2014 Jul 29. PMID: 25069544.
[8] Francis CY, Morris J, Whorwell PJ. The irritable bowel severity scoring system: a simple method of monitoring irritable bowel syndrome and its progress. Aliment Pharmacol Ther. 1997 Apr;11(2):395-402. doi: 10.1046/j.1365-2036.1997.142318000.x. PMID: 9146781.
[9] Drossman DA, Chang L, Bellamy N, Gallo-Torres HE, Lembo A, Mearin F, Norton NJ, Whorwell P. Severity in irritable bowel syndrome: a Rome Foundation Working Team report. Am J Gastroenterol. 2011 Oct;106(10):1749-59; quiz 1760. doi: 10.1038/ajg.2011.201. Epub 2011 Jul 12. PMID: 21747417.
[10] Blake MR, Raker JM, Whelan K. Validity and reliability of the Bristol Stool Form Scale in healthy adults and patients with diarrhoea-predominant irritable bowel syndrome. Aliment Pharmacol Ther. 2016 Oct;44(7):693-703. doi: 10.1111/apt.13746. Epub 2016 Aug 5. PMID: 27492648.
[11] Lovell RM, Ford AC. Global prevalence of and risk factors for irritable bowel syndrome: a meta-analysis. Clin Gastroenterol Hepatol. 2012 Jul;10(7):712-721.e4. doi: 10.1016/j.cgh.2012.02.029. Epub 2012 Mar 15. PMID: 22426087.
[12] Oka P, Parr H, Barberio B, Black CJ, Savarino EV, Ford AC. Global prevalence of irritable bowel syndrome according to Rome III or IV criteria: a systematic review and meta-analysis. Lancet Gastroenterol Hepatol. 2020 Oct;5(10):908-917. doi: 10.1016/52468-1253(20)30217-X. Epub 2020 Jul 20. Erratum in: Lancet Gastroenterol Hepatol. 2020 Dec;5(12):e8. PMID: 32702295.
[13] Lacy BE, Mearin F, Chang L, Chey WD, Lembo AJ, Simren J, Spiller R. Bowel disorders. Gastroenterology 2016, 150, 1393-1407.
[14] Barbara G, Feinle-Bisset C, Ghoshal UC, Quigley EM, Santos J, Vanner S, Vergnolle N, Zoetendal EG. The Intestinal Microenvironment and Functional Gastrointestinal Disorders. Gastroenterology. 2016 Feb 18:S0016-5085(16)00219-5. doi: 10.1053/j.gastro.2016.02.028. Epub ahead of print. PMID: 27144620.
[15] Tana C, Umesaki Y, Imaoka A, Handa T, Kanazawa M, Fukudo S. Altered profiles of intestinal microbiota and organic acids may be the origin of symptoms in irritable bowel syndrome. Neurogastroenterol Motil. 2010 May;22(5):512-9, e114-5. doi: 10.1111/j.1365-2982.2009.01427.x. Epub 2009 Nov 10. PMID: 19903265.
[16] Kerckhoffs AP, Samsom M, van der Rest ME, de Vogel J, Knol J, Ben-Amor K, Akkermans LM. Lower Bifidobacteria counts in both duodenal mucosa-associated and fecal microbiota in irritable bowel syndrome patients. World J Gastroenterol. 2009 Jun 21;15(23):2887-92. doi: 10.3748/wjg.15.2887. PMID: 19533811; PMCID: PMC2699007.
[17] Kassinen A, Krogius-Kurikka L, Mäkivuokko H, Rinttilä T, Paulin L, Corander J, Malinen E, Apajalahti J, Palva A. The fecal microbiota of irritable bowel syndrome patients differs significantly from that of healthy subjects. Gastroenterology. 2007 Jul;133(1):24-33. doi: 10.1053/j.gastro.2007.04.005. Epub 2007 Apr 14. PMID: 17631127.
[18] Tap J, Derrien M, Törnblom H, Brazeilles R, Cools-Portier S, Doré J, Störsrud S, Le Nevé B, Ohman L, Simrén M. Identification of an Intestinal Microbiota Signature Associated With Severity of Irritable Bowel Syndrome. Gastroenterology. 2017 Jan;152(1):111-123.e8. doi: 10.1053/j.gastro.2016.09.049. Epub 2016 Oct 7. PMID: 27725146.
[19] Malinen E, Krogius-Kurikka L, Lyra A, Nikkilä J, Jääskeläinen A, Rinttilä T, Vilpponen-Salmela T, von Wright AJ, Palva A. Association of symptoms with gastrointestinal microbiota in irritable bowel syndrome. World J Gastroenterol. 2010 Sep 28;16(36):4532-40. doi: 10.3748/wjg.v16.i36.4532. PMID: 20857523; PMCID: PMC2945484.
[20] Ceccherini C, Daniotti S, Bearzi C, Re I. Evaluating the Efficacy of Probiotics in IBS Treatment Using a Systematic Review of Clinical Trials and Multi-Criteria Decision Analysis. Nutrients. 2022 Jun 28;14(13):2689. doi: 10.3390/nu14132689. PMID: 35807868; PMCID: PMC9268703.
[21] van der Geest AM, Schukking I, Brummer RJM, van de Burgwal LHM, Larsen OFA. Comparing probiotic and drug interventions in irritable bowel syndrome: a meta-analysis of randomised controlled trials. Benef Microbes. 2022 Aug 3;13(3):183-194. doi: 10.3920/BM2021.0123. Epub 2022 Jul 18. PMID: 35848115.
[22] Dale HF, Rasmussen SH, Asiller ÖÖ, Lied GA. Probiotics in Irritable Bowel Syndrome: An Up-to-Date Systematic Review. Nutrients. 2019 Sep 2;11(9):2048. doi: 10.3390/nu11092048. PMID: 31480656; PMCID: PMC6769995.
[23] Xie P, Luo M, Deng X, Fan J, Xiong L. Outcome-Specific Efficacy of Different Probiotic Strains and Mixtures in Irritable Bowel Syndrome: A Systematic Review and Network Meta-Analysis. Nutrients. 2023 Sep 4;15(17):3856. doi: 10.3390/nu15173856. PMID: 37686889; PMCID: PMC10490209.
[24] Zhang T, Zhang C, Zhang J, Sun F, Duan L. Efficacy of Probiotics for Irritable Bowel Syndrome: A Systematic Review and Network Meta-Analysis. Front Cell Infect Microbiol. 2022 Apr 1;12:859967. doi: 10.3389/fcimb.2022.859967. PMID: 35433498; PMCID: PMC9010660.
[25] Niu HL, Xiao JY. The efficacy and safety of probiotics in patients with irritable bowel syndrome: Evidence based on 35 randomized controlled trials. Int J Surg. 2020 Mar;75:116-127. doi: 10.1016/j.ijsu.2020.01.142. Epub 2020 Jan 31. PMID: 32014597.
[26] Liang D, Longgui N, Guoqiang X. Efficacy of different probiotic protocols in irritable bowel syndrome: A network meta-analysis. Medicine (Baltimore). 2019 Jul;98(27):e16068. doi: 10.1097/MD.0000000000016068. PMID: 31277101; PMCID: PMC6635271.
[27] Harris LA, Baffy N. Modulation of the gut microbiota: a focus on treatments for irritable bowel syndrome. Postgrad Med. 2017 Nov;129(8):872-888. doi: 10.1080/00325481.2017.1383819. Epub 2017 Oct 13. PMID: 28936910.
[28] Didari T, Mozaffari S, Nikfar S, Abdollahi M. Effectiveness of probiotics in irritable bowel syndrome: Updated systematic review with meta-analysis. World J Gastroenterol. 2015 Mar 14;21(10):3072-84. doi: 10.3748/wjg.v21.i10.3072. PMID: 25780308; PMCID: PMC4356930.
[29] Gracie DJ, Ford AC. Symbiotics in irritable bowel syndrome--better than probiotics alone? Curr Opin Clin Nutr Metab Care. 2015 Sep;18(5):485-9. doi: 10.1097/MCO.0000000000000199. PMID: 26107141.
[30] Chong PP, Chin VK, Looi CY, Wong WF, Madhavan P, Yong VC. The Microbiome and Irritable Bowel Syndrome - A Review on the Pathophysiology, Current Research and Future Therapy. Front Microbiol. 2019 Jun 10;10:1136. doi: 10.3389/fmicb.2019.01136. Erratum in: Front Microbiol. 2019 Aug 13;10:1870. PMID: 31244784; PMCID: PMC6579922.
[31] Chlebicz-Wójcik A, Śliżewska K. Probiotics, Prebiotics, and Synbiotics in the Irritable Bowel Syndrome Treatment: A Review. Biomolecules. 2021 Aug 4;11(8):1154. doi: 10.3390/biom11081154. PMID: 34439821; PMCID: PMC8412098.
[32] Sen S, Mullan MM, Parker TJ, Woolner JT, Tarry SA, Hunter JO. Effect of Lactobacillus plantarum 299v on colonic fermentation and symptoms of irritable bowel syndrome. Dig Dis Sci. 2002 Nov;47(11):2615-20. doi: 10.1023/a:1020597001460. PMID: 12452404.
[33] Pedersen SM, Nielsen NC, Andersen HJ, Olsson J, Simrén M, Ohman L, Svensson U, Malmendal A, Bertram HC. The serum metabolite response to diet intervention with probiotic acidified milk in irritable bowel syndrome patients is indistinguishable from that of non-probiotic acidified milk by 1H NMR-based metabonomic analysis. Nutrients. 2010 Nov;2(11):1141-55. doi: 10.3390/nu2111141. Epub 2010 Nov 23. PMID: 22254002; PMCID: PMC3257620.
[34] Simrén M, Ohman L, Olsson J, Svensson U, Ohlson K, Posserud I, Strid H. Clinical trial: the effects of a fermented milk containing three probiotic bacteria in patients with irritable bowel syndrome - a randomized, double-blind, controlled study. Aliment Pharmacol Ther. 2010 Jan 15;31(2):218-27. doi: 10.1111/j.1365-2036.2009.04183.x. Epub 2009 Oct 26. PMID: 19863495.
[35] Søndergaard B, Olsson J, Ohlson K, Svensson U, Bytzer P, Ekesbo R. Effects of probiotic fermented milk on symptoms and intestinal flora in patients with irritable bowel syndrome: a randomized, placebo-controlled trial. Scand J Gastroenterol. 2011 Jun;46(6):663-72. doi: 10.3109/00365521.2011.565066. Epub 2011 Mar 28. PMID: 21443416.
[36] Amirimani B, Nikfam S, Albaji M, Vahedi S, Nasseri-Moghaddam S, Sharafkhah M, Ansari R, Vahedi H. Probiotic vs. Placebo in Irritable Bowel Syndrome:A Randomized Controlled Trial. Middle East J Dig Dis. 2013 Apr;5(2):98-102. PMID: 24829677; PMCID: PMC3990144.
[37] Roberts LM, McCahon D, Holder R, Wilson S, Hobbs FD. A randomised controlled trial of a probiotic 'functional food' in the management of irritable bowel syndrome. BMC Gastroenterol. 2013 Mar 7;13:45. doi: 10.1186/1471-230X-13-45. PMID: 23496803; PMCID: PMC3605320.
[38] Cremon C, Guglielmetti S, Gargari G, Taverniti V, Castellazzi AM, Valsecchi C, Tagliacarne C, Fiore W, Bellini M, Bertani L, Gambaccini D, Cicala M, Germanà B, Vecchi M, Pagano I, Barbaro MR, Bellacosa L, Stanghellini V, Barbara G. Effect of Lactobacillus paracasei CNCM I-1572 on symptoms, gut microbiota, short chain fatty acids, and immune activation in patients with irritable bowel syndrome: A pilot randomized clinical trial. United European Gastroenterol J. 2018 May;6(4):604-613. doi: 10.1177/2050640617736478. Epub 2017 Oct 8. PMID: 29881616; PMCID: PMC5987284.
[39] Dapoigny M, Piche T, Ducrotte P, Lunaud B, Cardot JM, Bernalier-Donadille A. Efficacy and safety profile of LCR35 complete freeze-dried culture in irritable bowel syndrome: a randomized, double-blind study. World J Gastroenterol. 2012 May 7;18(17):2067-75. doi: 10.3748/wjg.v18.i17.2067. PMID: 22563194; PMCID: PMC3342605.
[40] Ligaarden SC, Axelsson L, Naterstad K, Lydersen S, Farup PG. A candidate probiotic with unfavourable effects in subjects with irritable bowel syndrome: a randomised controlled trial. BMC Gastroenterol. 2010 Feb 10;10:16. doi: 10.1186/1471-230X-10-16. PMID: 20144246; PMCID: PMC2831047.
[41] Bucci C, Tremolaterra F, Gallotta S, Fortunato A, Cappello C, Ciacci C, lovino P. A pilot study on the effect of a symbiotic mixture in irritable bowel syndrome: an open-label, partially controlled, 6-month extension of a previously published trial. Tech Coloproctol. 2014 Apr;18(4):345-53. doi: 10.1007/s10151-013-1055-2. Epub 2013 Aug 7. PMID: 23922211.
[42] Cappello C, Tremolaterra F, Pascariello A, Ciacci C, lovino P. A randomised clinical trial (RCT) of a symbiotic mixture in patients with irritable bowel syndrome (IBS): effects on symptoms, colonic transit and quality of life. Int J Colorectal Dis. 2013 Mar;28(3):349-58. doi: 10.1007/s00384-012-1552-1. Epub 2012 Aug 12. PMID: 22885882; PMCID: PMC3587687.
[43] Shavakhi A, Minakari M, Farzamnia S, Peykar MS, Taghipour G, Tayebi A, Hashemi H, Shavakhi S. The effects of multi-strain probiotic compound on symptoms and quality-of-life in patients with irritable bowel syndrome: A randomized placebo-controlled trial. Adv Biomed Res. 2014 Jun 25;3:140. doi: 10.4103/2277-9175.135157. PMID: 25161987; PMCID: PMC4139977.
[44] IBS questionnaire for HCP. Available online: https://www.worldgastroenterology.org/UserFiles/file/wdhd-2009-test-for-diagnosing-ibs.pdf (accessed on 18.07.2023)
[45] Gordon S, Ameen V, Bagby B, Shahan B, Jhingran P, Carter E. Validation of irritable bowel syndrome Global Improvement Scale: an integrated symptom end point for assessing treatment efficacy. Dig Dis Sci. 2003 Jul;48(7):1317-23. doi: 10.1023/a:1024159226274. PMID: 12870789.
[46] Passos MC, Lembo AJ, Conboy LA, Kaptchuk TJ, Kelly JM, Quilty MT, Kerr CE, Jacobson EE, Hu R, Friedlander E, Drossman DA. Adequate relief in a treatment trial with IBS patients: a prospective assessment. Am J Gastroenterol. 2009 Apr;104(4):912-9. doi: 10.1038/ajg.2009.13. Epub 2009 Mar 17. PMID: 19293784; PMCID: PMC2707022.

## Claims

1. A pharmaceutical composition for use in a method for the treatment of irritable bowel syndrome (IBS), the pharmaceutical composition comprising a probiotic comprising or consisting of the following bacterial strains:
Lactobacillus helveticus;
Lacticaseibacillus rhamnosus;
Lacticaseibacillus casei;
Lactiplantibacillus plantarum;
Lactococcus lactis;
Bifidobacterium longum;
Bifidobacterium breve;
Bifidobacterium bifidum; and
Streptococcus thermophilus.

2. The pharmaceutical composition for use according to claim 1, wherein the method comprises administering the pharmaceutical composition to a subject with IBS-D (irritable bowel syndrome with diarrhea) or IBS-C (irritable bowel syndrome with constipation) and/or wherein the IBS is IBS-D and/or IBS-C.

3. The composition for use according to claim 1 or 2, wherein the method comprises administering the pharmaceutical composition to a subject with severe IBS and/or wherein the IBS is severe IBS.

4. The composition for use according to claim 1 or 2, wherein the method comprises administering the pharmaceutical composition to a subject with moderate IBS and/or wherein the IBS is moderate IBS.

5. The pharmaceutical composition for use according any one of the preceding claims for the treatment of abdominal pain and/or bloating and/or stool pressure and/or incomplete evacuation of stool associated with IBS.

6. The pharmaceutical composition for use according to any one of the preceding claims, wherein the probiotic comprises or is composed of:
15% to 25% Lactobacillus helveticus;
7% to 13% Lacticaseibacillus rhamnosus;
3% to 7% Lacticaseibacillus casei;
3% to 7% Lactiplantibacillus plantarum;
15% to 25% Lactococcus lactis;
11% to 19% Bifidobacterium longum;
7% to 13% Bifidobacterium breve;
3% to 7% Bifidobacterium bifidum; and
7% to 13% Streptococcus thermophilus,
wherein % refers to % of total CFU (colony forming units) contained in the probiotic and/or the pharmaceutical composition.

7. The pharmaceutical composition for use according to any one of the preceding claims, wherein the probiotic comprises or is composed of:
7.00 × 10⁸ to 11.00 × 10⁸ CFU Lactobacillus helveticus;
3.50 × 10⁸ to 5.50 × 10⁸ CFU Lacticaseibacillus rhamnosus;
1.75 × 10⁸ to 2.75 × 10⁸ CFU Lacticaseibacillus casei;
1.75 × 10⁸ to 2.75 × 10⁸ CFU Lactiplantibacillus plantarum;
7.00 × 10⁸ to 11.00 × 10⁸ CFU Lactococcus lactis;
5.25 × 10⁸ to 8.25 × 10⁸ CFU Bifidobacterium longum;
3.50 × 10⁸ to 5.50 × 10⁸ CFU Bifidobacterium breve;
1.75 × 10⁸ to 2.75 × 10⁸ CFU Bifidobacterium bifidum; and
3.50 × 10⁸ to 5.50 × 10⁸ CFU Streptococcus thermophilus.

8. The pharmaceutical composition for use according to any one of the preceding claims, wherein the lactobacillus helveticus is lactobacillus helveticus SP 27, the lacticaseibacillus rhamnosus is lacticaseibacillus rhamnosus Lr-32, the lacticaseibacillus casei is lacticaseibacillus casei Lc-11, the lactiplantibacillus plantarum is lactiplantibacillus plantarum Lp-115, the lactococcus lactis is lactococcus lactis LI-23, the bifidobacterium longum is bifidobacterium longum BI-05 and/or bifidobacterium longum ES1, the bifidobacterium breve is bifidobacterium breve Bb-03, the bifidobacterium bifidum is bifidobacterium bifidum Bb-02, and/or the streptococcus thermophilus is streptococcus thermophilus St-21.

9. The pharmaceutical composition for use according to any one of the preceding claims, further comprising at least one prebiotic.

10. The pharmaceutical composition for use according to claim 9, wherein the at least one prebiotic is fructooligosaccharides (FOS) and/or wherein the pharmaceutical composition comprises 20 mg to 100 mg of the at least one prebiotic.

11. The pharmaceutical composition for use according to any one of the preceding claims, wherein the pharmaceutical composition is in oral dosage form.

12. The pharmaceutical composition for use according to any one of the preceding claims, wherein the probiotic and optionally the prebiotic are contained in a capsule.

13. The pharmaceutical composition for use according to claim 12, wherein the capsule comprises or consists of hydroxypropyl methylcellulose, optionally wherein the capsule comprises an enteric coating.

14. The pharmaceutical composition for use according to any one of the preceding claims, wherein the method comprises orally administering the pharmaceutical composition to a subject.

15. The pharmaceutical composition for use according to any one of the preceding claims, wherein the method comprises administering the pharmaceutical composition to a subject once daily.
